# EUROPEAN PATENT SPECIFICATION

(11) **EP 4 236 932 B1**
(45) Date of publication and mention of the grant of the patent: **07.01.2026**
(21) Application number: 21887770.2
(22) Date of filing: 02.11.2021
(51) Int. Cl.: A61K 9/00, A61K 31/27, A61K 31/498, A61K 47/38, A61K 31/165, A61K 31/4164, A61K 31/439

(54) **DEGRADANT COMPOUND IN A MEDICAMENT**
ABBAUENDE VERBINDUNG IN EINEM MEDIKAMENT
COMPOSÉ DE DÉGRADATION DANS UN MÉDICAMENT

(30) Priority: 02.11.2020 US 202063108720 P; 17.03.2021 US 202163162367 P; 18.10.2021 US 202163257024 P
(43) Date of publication of application: 06.09.2023
(73) Proprietor: Visus Therapeutics, Inc., Seattle, Washington 98109 (US)
(72) Inventor: SCHIFFMAN, Rhett Mead, Laguna Beach, California 92651 (US); FIRESTONE, Bruce Alan, Irvine, California 92603 (US)
(74) Representative: Carpmaels & Ransford LLP
(86) International application number: PCT/US2021/057720
(87) International publication number: WO 2022/094462

(56) References cited:
- WO-A1-2016/205068
- WO-A1-2017/160548
- WO-A1-2019/104191
- WO-A1-2020/252061
- US-A1- 2012 329 805
- Declaration of the inventor, Schiffman, dated 25.10.2024
- ABDELKADER ET AL.: "Clinical outcomes of combined versus separate carbachol and brimonidine drops in correcting presbyopia", EYE AND VISION, vol. 3, 5 December 2016 (2016-12-05), pages 1 - 6, XP055869051, DOI: 10.1186/s40662-016-0065-3

## Description

### FIELD AND BACKGROUND OF THE INVENTION

The present disclosure relates to ophthalmic formulations comprising carbachol, brimonidine, and less than 5 wt % of one or more impurities. The formulations disclosed may be useful in treating ophthalmic conditions such as presbyopia.

Presbyopia is typically age-related eye deterioration. Young, properly functioning, eyes are able to see at near distances, an ability that deteriorates as one ages. Presbyopia normally develops as a person ages, and is associated with a natural progressive loss of accommodation. A presbyopic eye loses the ability to rapidly and easily focus on objects at near distances. Presbyopia progresses over the lifetime of an individual, usually becoming noticeable after the age of 45 years. By the age of 65 years, the crystalline lens has often lost almost all elastic properties and has only limited ability to change shape.

Use of over the counter reading glasses is a very common way of addressing the vision problems associated with presbyopia. Reading glasses allow the eye to focus on near objects and maintain a clear image. This approach is similar to that of treating hyperopia, or farsightedness.

Many presbyopes are also prescribed bi-focal eyeglasses, where one portion of the lens is corrected for distance vision and another portion of the lens is corrected for near vision. When peering down through the bifocals, the individual looks through the portion of the lens corrected for near vision. When viewing distant objects, the individual looks higher, through the portion of the bi-focals corrected for distance vision. Contact lenses and intra-ocular lenses (IOLs) have also been used to treat presbyopia, for example, by relying on monovision (where one eye is corrected for distance-vision, while the other eye is corrected for near-vision) or bilateral correction with either bi-focal or multi-focal lenses. Laser ablation has also been used to treat presbyopia. All these procedures seek to correct the problem for long term purposes using drastic steps (surgery, laser ablation, etc) or require wearing corrective lenses.

There remains a need for new ways of ameliorating or reducing presbyopia for patients that do not wish to undergo surgery (IOLs, laser ablation, etc.) or use corrective glasses. For people who use corrective lenses, there remains a need to temporarily treat presbyopia without the use of corrective lenses.

As with any formulation designed for treatment of a subject in need thereof, it is important to identify and quantify the presence of any impurities in the formulation. In particular, formulations comprising two or more active agents may result in unexpected reactivity between said agents, resulting in the formation of impurity species. Thus, there is a need to identify and quantify the presence of any impurity species in ophthalmic formulations comprising carbachol and brimonidine.

WO 2020/252061 relates to the use of topical carbachol in combination with brimonidine which is said to create optically beneficial miosis to temporarily treat presbyopia. A pharmaceutical formulation is used comprising a therapeutically effective amount of carbachol, or a pharmaceutically acceptable salt thereof, in combination with brimonidine, or a pharmaceutically acceptable salt thereof, specifically combined with permeation enhancers and excipients, and is said to increase efficacy and reduce ocular surface toxicity and improve tolerability.

Any references in the description to methods of treatment according to the invention refer to the compounds, pharmaceutical compositions and medicaments of the present invention for use in that method of treatment.

### BRIEF SUMMARY OF THE INVENTION

The present invention is defined by the appended claims, and relates to an ophthalmic formulation comprising 2.75 wt% carbachol, or a pharmaceutically acceptable salt thereof, 0.1 wt% brimonidine, or a pharmaceutically acceptable salt thereof, 0.2 wt% hydroxypropylmethyl cellulose (HPMC), and from 0.05 wt % to 1 wt % of one or more buffers, wherein the pH is 7.4, and wherein the formulation comprises: (i) less than 5 wt % impurity A after storage at 40 °C and not more than (NMT) 25% relative humidity for 5 months, wherein impurity A has the structure: 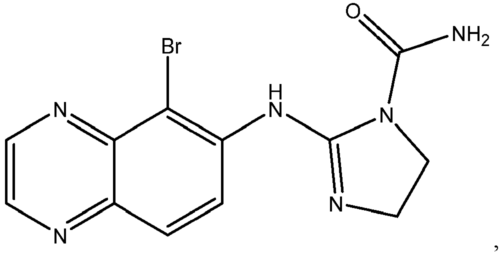 or a tautomer thereof, wherein the concentration of impurity A is measured by high-performance liquid chromatography (HPLC) in pH 3.5 potassium octane sulfonate buffer:acetonitrile (64:36 v/v) and impurity A has a relative retention time (RRT) of 0.9 relative to brimonidine; and/or (ii) less than 2 wt % impurity B after storage at 40 °C and not more than (NMT) 25% relative humidity for 5 months, wherein impurity B has the structure: 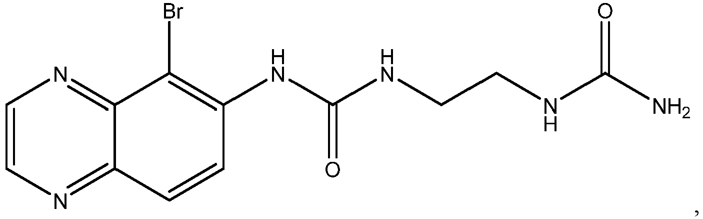 or a tautomer thereof, wherein the concentration of impurity B is measured by high-performance liquid chromatography (HPLC) in pH 3.5 potassium octane sulfonate buffer:acetonitrile (64:36 v/v) and impurity B has a relative retention time (RRT) of 0.67 relative to brimonidine.

In some aspects, the ophthalmic formulation is stored at 40 °C and not more than (NMT) 25% relative humidity for about 5 months.

In some aspects, the ophthalmic formulation comprises less than 3 wt % impurity A after storage at 40 °C and NMT 25% relative humidity for about 3 months.

In some aspects, the ophthalmic formulation comprises less than 0.25 wt % impurity A.

In some aspects, the ophthalmic formulation comprises less than 1 wt % impurity A after storage at 25 °C and 40% relative humidity for about 5 months.

In some aspects, the ophthalmic formulation comprises less than 0.1 wt % impurity B after storage for about 5 months.

In some aspects, the ophthalmic formulation comprises less than 2 wt % impurity B after storage at 40 °C and NMT 25% relative humidity for about 5 months.

In some aspects, the ophthalmic formulation comprises less than 0.25 wt % impurity B after storage at 25 °C and 40% relative humidity for about 3 months.

In some aspects, an ophthalmic formulation described herein comprises one or more buffers selected from the group consisting of acetate buffer, borate buffer, borate citrate buffer, carbonate buffer, citrate buffer, lactate buffer, and phosphate buffer.

In some aspects, an ophthalmic formulation described herein comprises one or more buffers that is a phosphate buffer.

In some aspects, the phosphate buffer comprises sodium phosphate monobasic monohydrate and sodium phosphate dibasic heptahydrate.

In some aspects, an ophthalmic formulation described herein comprises from about 0.0025 wt % to about 0.02 wt % benzalkonium chloride.

In some aspects, an ophthalmic formulation described herein does not contain EDTA.

The present invention also relates to the use of ophthalmic formulations of the invention for ameliorating or reducing presbyopia in at least one eye of a subject.

### DETAILED DESCRIPTION OF THE INVENTION

### Definitions

Unless defined otherwise, all technical and scientific terms used herein have the same meaning as commonly understood by one of ordinary skill in the art to which this disclosure belongs. In case of conflict, the present application including the definitions will control. Unless otherwise required by context, singular terms shall include pluralities and plural terms shall include the singular.

Although methods and materials similar or equivalent to those described herein can be used in practice or testing of the present disclosure, suitable methods and materials are described below. The materials, methods and examples are illustrative only and are not intended to be limiting. Other features and advantages of the disclosure will be apparent from the detailed description and from the claims.

In order to further define this disclosure, the following terms and definitions are provided.

The singular forms "a," "an" and "the" include plural referents unless the context clearly dictates otherwise. The terms "a" (or "an"), as well as the terms "one or more," and "at least one" can be used interchangeably herein. In certain aspects, the term "a" or "an" means "single." In other aspects, the term "a" or "an" includes "two or more" or "multiple."

The term "about" is used herein to mean approximately, roughly, around, or in the regions of. When the term "about" is used in conjunction with a numerical range, it modifies that range by extending the boundaries above and below the numerical values set forth. In general, the term "about" is used herein to modify a numerical value above and below the stated value by a variance of 10 percent, up or down (higher or lower).

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term "pharmaceutically acceptable" as used herein refers to those compounds, materials, compositions, formulations, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "excipient" refers to any substance, not itself a therapeutic agent, which may be used in a composition for delivery of an active therapeutic agent to a subject or combined with an active therapeutic agent (e.g., to create a pharmaceutical composition) to improve its handling or storage properties or to permit or facilitate formation of a dose unit of the composition. The excipient can be an inert substance, an inactive substance, and/or a not medicinally active substance.

The terms "effective amount" or "pharmaceutically effective amount" or "therapeutically effective amount" as used herein refer to the amount or quantity of a drug or pharmaceutically active substance which is sufficient to elicit the required or desired therapeutic response, or in other words, the amount which is sufficient to elicit an appreciable biological response when administered to a patient.

"Administration", or "to administer" means the step of giving (i.e. providing) a pharmaceutical composition to a subject. The pharmaceutical compositions disclosed herein can be "locally administered", that is administered at or in the vicinity of the site at which a therapeutic result or outcome is desired. For example to treat an ocular condition such as corneal pain, topical administration, directly to the eye of a subject, of an ophthalmic formulation can be carried out, and is an example of local administration.

The term "pharmaceutically acceptable salts" is art-recognized, and includes relatively non-toxic, inorganic and organic acid addition salts of compounds and relatively non-toxic, inorganic and organic base addition salts of compounds.

Inorganic acids which may be used to prepare pharmaceutically acceptable salts include, but are not limited to, hydrochloric acid, phosphoric acid, sulfuric acid, hydrobromic acid, hydroiodic acid, phosphorous acid and the like. Organic acids which may be used to prepare pharmaceutically acceptable salts include, without limitation, aliphatic mono- and dicarboxylic acids, such as tartaric acid, oxalic acid, carbonic acid, citric acid, succinic acid, phenyl-heteroatom-substituted alkanoic acids, aliphatic and aromatic sulfuric acids and the like. Pharmaceutically acceptable salts prepared from inorganic or organic acids thus include, but are not limited to, hydrochloride, hydrobromide, nitrate, sulfate, pyrosulfate, bisulfate, sulfite, bisulfite, phosphate, monohydrogenphosphate, dihydrogenphosphate, metaphosphate, pyrophosphate, hydroiodide, hydrofluoride, acetate, propionate, formate, oxalate, tartrate, citrate, lactate, p-toluenesulfonate, methanesulfonate, and maleate. Suitable pharmaceutically acceptable salts may also be formed by reacting the active components with an organic base such as methylamine, ethylamine, ethanolamine, lysine, ornithine and the like. Pharmaceutically acceptable salts include the salts formed between carboxylate or sulfonate groups that may be found on some of the active components and inorganic cations, such as sodium, potassium, ammonium, or calcium, or such organic cations as isopropylammonium, trimethylammonium, tetramethylammonium, and imidazolium. All of these salts may be prepared by conventional means from the active components of the invention by reacting, for example, the appropriate acid or base with the active components of the invention.

The term "unit dosage form" or "unit dose composition" as used herein refers to a quantity of a compound, such as a drop or a droplet, said quantity being such that one or more predetermined units may be provided as a single therapeutic administration.

As used herein, "treat," "treatment", and "treating" refer to the reduction or amelioration of the progression, severity, and/or duration of a given disease resulting from the administration of one or more therapies (including, but not limited to, the administration of an ophthalmic formulation). In certain aspects, the terms refer to the reduction of pain associated with one or more diseases or conditions.

The term "wt %" or "weight/volume" as used herein refers to the ratio between two components with respect to volume. For example, a 5 wt % ethanol in water solution would represent a solution comprising 5 g ethanol for every 100 mL water.

The term "mg/mL" as used herein refers to the ratio between a solute, generally an active pharmaceutical ingredient or excipient, and a solvent, generally but not necessarily water. For example, a 50 mg/mL sodium chloride aqueous solution would represent a solution comprising 50 mg sodium chloride for every 1 mL water.

The term "stable" is used herein to describe a composition in which most or all of the active pharmaceutical ingredient does not degrade or transform over a specific time period and under specific conditions.

### Impurity Compounds

The present disclosure provides compounds (not claimed) that may be formed as impurities in formulations comprising carbachol and brimonidine.

In some aspects, the compound is 2-((5-bromoquinoxalin-6-yl)amino)-4,5-dihydro-1H-imidazole-1-carboxamide ("impurity A"), having the structure: 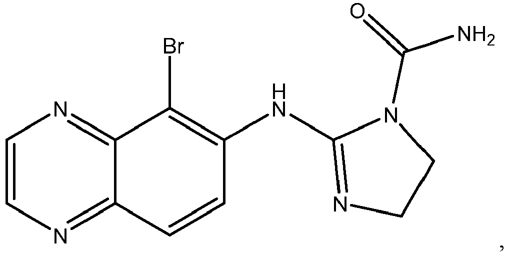 or a tautomer thereof.

In some aspects, impurity A has a relative retention time (RRT) of about 0.9 relative to brimonidine when measured by high performance liquid chromatography (HPLC) in pH 3.5 potassium octane sulfonate buffer:acetonitrile (64:36 v/v).

In some aspects, the compound is 1-(5-bromoquinoxalin-6-yl)-3-(2-ureidoethyl)urea ("impurity B"), having the structure: 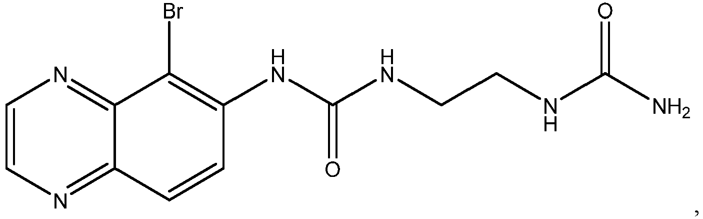 or a tautomer thereof.

In some aspects, impurity B has a RRT of about 0.67 relative to brimonidine when measured by HPLC in pH 3.5 potassium octane sulfonate buffer:acetonitrile (64:36 v/v).

### Ophthalmic Formulations

The present disclosure also provides ophthalmic formulations comprising 2.75 wt% carbachol, or a pharmaceutically acceptable salt thereof, 0.1 wt% brimonidine, or a pharmaceutically acceptable salt thereof, and 0.2 wt% hydroxypropylmethyl cellulose (HPMC), and from 0.05 wt % to 1 wt % of one or more buffers, wherein the pH is 7.4, and wherein the formulation comprises (i) less than 5 wt % impurity A after storage at 40 °C and not more than (NMT) 25% relative humidity for 5 months, wherein the concentration of impurity A is measured by high-performance liquid chromatography (HPLC) in pH 3.5 potassium octane sulfonate buffer:acetonitrile (64:36 v/v) and impurity A has a relative retention time (RRT) of 0.9 relative to brimonidine, and/or (ii) less than 2 wt % impurity B after storage at 40 °C and not more than (NMT) 25% relative humidity for 5 months, wherein the concentration of impurity B is measured by high-performance liquid chromatography (HPLC) in pH 3.5 potassium octane sulfonate buffer:acetonitrile (64:36 v/v) and impurity B has a relative retention time (RRT) of 0.67 relative to brimonidine.

In some aspects, the pharmaceutically acceptable salt of brimonidine is brimonidine tartrate.

In some aspects, the viscosity of the ophthalmic formulation is from about 1 cPs to about 5 cPs, from about 1 cPs to about 10 cPs, from about 1 cPs to about 15 cPs, from about 1 cPs to about 20 cPs, from about 1 cPs to about 30 cPs, from about 1 cPs to about 40 cPs, from about 1 cPs to about 50 cPs, from about 1 cPs to about 60 cPs, from about 1 cPs to about 80 cPs, from about 1 cPs to about 100 cPs, from about 1 cPs to about 125 cPs, from about 1 cPs to about 150 cPs, from about 1 cPs to about 175 cPs, from about 1 cPs to about 200 cPs, from about 1 cPs to about 400 cPs, from about 5 cPs to about 10 cPs, from about 5 cPs to about 15 cPs, from about 5 cPs to about 20 cPs, from about 5 cPs to about 30 cPs, from about 5 cPs to about 40 cPs, from about 5 cPs to about 50 cPs, from about 5 cPs to about 60 cPs, from about 5 cPs to about 80 cPs, from about 5 cPs to about 100 cPs, from about 5 cPs to about 125 cPs, from about 5 cPs to about 150 cPs, from about 5 cPs to about 175 cPs, from about 5 cPs to about 200 cPs, from about 5 cPs to about 400 cPs, from about 10 cPs to about 15 cPs, from about 10 cPs to about 20 cPs, from about 10 cPs to about 30 cPs, from about 10 cPs to about 40 cPs, from about 10 cPs to about 50 cPs, from about 10 cPs to about 60 cPs, from about 10 cPs to about 80 cPs, from about 10 cPs to about 100 cPs, from about 10 cPs to about 125 cPs, from about 10 cPs to about 150 cPs, from about 10 cPs to about 175 cPs, from about 10 cPs to about 200 cPs, from about 10 cPs to about 400 cPs, from about 15 cPs to about 20 cPs, from about 15 cPs to about 30 cPs, from about 15 cPs to about 40 cPs, from about 15 cPs to about 50 cPs, from about 15 cPs to about 60 cPs, from about 15 cPs to about 80 cPs, from about 15 cPs to about 100 cPs, from about 15 cPs to about 125 cPs, from about 15 cPs to about 150 cPs, from about 15 cPs to about 175 cPs, from about 15 cPs to about 200 cPs, from about 15 cPs to about 400 cPs, from about 20 cPs to about 30 cPs, from about 20 cPs to about 40 cPs, from about 20 cPs to about 50 cPs, from about 20 cPs to about 60 cPs, from about 20 cPs to about 80 cPs, from about 20 cPs to about 100 cPs, from about 20 cPs to about 125 cPs, from about 20 cPs to about 150 cPs, from about 20 cPs to about 175 cPs, from about 20 cPs to about 200 cPs, from about 20 cPs to about 400 cPs, from about 30 cPs to about 40 cPs, from about 30 cPs to about 50 cPs, from about 30 cPs to about 60 cPs, from about 30 cPs to about 80 cPs, from about 30 cPs to about 100 cPs, from about 30 cPs to about 125 cPs, from about 30 cPs to about 150 cPs, from about 30 cPs to about 175 cPs, from about 30 cPs to about 200 cPs, from about 30 cPs to about 400 cPs, from about 40 cPs to about 50 cPs, from about 40 cPs to about 60 cPs, from about 40 cPs to about 80 cPs, from about 40 cPs to about 100 cPs, from about 40 cPs to about 125 cPs, from about 40 cPs to about 150 cPs, from about 40 cPs to about 175 cPs, from about 40 cPs to about 200 cPs, from about 40 cPs to about 400 cPs, from about 50 cPs to about 60 cPs, from about 50 cPs to about 80 cPs, from about 50 cPs to about 100 cPs, from about 50 cPs to about 125 cPs, from about 50 cPs to about 150 cPs, from about 50 cPs to about 175 cPs, from about 50 cPs to about 200 cPs, from about 50 cPs to about 400 cPs, from about 60 cPs to about 80 cPs, from about 60 cPs to about 100 cPs, from about 60 cPs to about 125 cPs, from about 60 cPs to about 150 cPs, from about 60 cPs to about 175 cPs, from about 60 cPs to about 200 cPs, from about 60 cPs to about 400 cPs, from about 80 cPs to about 100 cPs, from about 80 cPs to about 125 cPs, from about 80 cPs to about 150 cPs, from about 80 cPs to about 175 cPs, from about 80 cPs to about 200 cPs, from about 80 cPs to about 400 cPs, from about 100 cPs to about 125 cPs, from about 100 cPs to about 150 cPs, from about 100 cPs to about 175 cPs, from about 100 cPs to about 200 cPs, from about 100 cPs to about 400 cPs, from about 125 cPs to about 150 cPs, from about 125 cPs to about 175 cPs, from about 125 cPs to about 200 cPs, from about 125 cPs to about 400 cPs, from about 150 cPs to about 175 cPs, from about 150 cPs to about 200 cPs, from about 150 cPs to about 400 cPs, from about 175 cPs to about 200 cPs, from about 175 cPs to about 400 cPs, or from about 200 cPs to about 400 cPs. In some aspects, the viscosity of the ophthalmic formulation is from about 10 cPs to about 30 cPs. (1 cps = 1 mPa·s).

In some aspects, the ophthalmic formulation has a viscosity of about 1 cPs, about 5 cPs, about 10 cPs, about 15 cPs, about 20 cPs, about 30 cPs, about 40 cPs, about 50 cPs, about 60 cPs, about 80 cPs, about 100 cPs, about 125 cPs, about 150 cPs, about 175 cPs, about 200 cPs, or about 400 cPs. In some aspects, the ophthalmic formulation has a viscosity of about 30 cPs.

The ophthalmic formulation comprises one or more buffers. Non-limiting examples of buffers include acetate buffer, borate buffer, borate citrate buffer, citrate buffer, lactate buffer, phosphate buffer, succinate buffer, borate-polyol complex buffer, carbonate buffer, an organic buffer, an amino acid buffer, and combinations thereof. In some aspects, the ophthalmic formulation comprises one or more buffers that is a phosphate buffer.

In some aspects, the phosphate buffer comprises phosphoric acid; alkali metal phosphates such as disodium hydrogen phosphate, sodium phosphate monobasic monohydrate, sodium dihydrogen phosphate, sodium phosphate dibasic heptahydrate, trisodium phosphate, dipotassium hydrogen phosphate, potassium dihydrogen phosphate, and tripotassium phosphate; alkaline earth metal phosphates such as calcium phosphate, calcium hydrogen phosphate, calcium dihydrogen phosphate, monomagnesium phosphate, dimagnesium phosphate (magnesium hydrogen phosphate), and trimagnesium phosphate; ammonium phosphates such as diammonium hydrogen phosphate and ammonium dihydrogen phosphate; or a combination thereof. In some aspects, the phosphate buffer comprises one or more anhydrides. In some aspects, the phosphate buffer comprises one or more hydrates.

Organic buffers include, but are not limited to, Good's Buffer, such as for example 2-(N-morpholino)ethanesulfonic acid (MES), N-(2-Acetamido)iminodiacetic acid, N-(Carbamoylmethyl)iminodiacetic acid (ADA), piperazine-N,N'-bis(2-ethanesulfonic acid (PIPES), N-(2-acetamido)-2-aminoethanesulfonic acid (ACES), β-Hydroxy-4-morpholinepropanesulfonic acid, 3-Morpholino-2-hydroxypropanesulfonic acid (MOPSO), cholamine chloride, 3-(N-morpholino)propansulfonic acid (MOPS), N,N-bis(2-hydroxyethyl)-2-aminoethanesulfonic acid (BES), 2-[(2-Hydroxy-1,1-bis(hydroxymethyl)ethyl)amino]ethanesulfonic acid (TES), 4-(2-hydroxyethyl)-1-piperazineethanesulfonic acid (HEPES), 3-(N,N-Bis[2-hydroxyethyl]amino)-2-hydroxypropanesulfonic acid (DIPSO), acetamidoglycine, 3-{[1,3-Dihydroxy-2-(hydroxymethyl)-2-propanyl]amino}-2-hydroxy-1-propanesulfonic acid (TAPSO), piperazine-1,4,-bis (2-hydroxypropanesulphonic acid) (POPSO), 4-(2-hydroxyethyl)piperazine-1-(2-hydroxypropanesulfonic acid) hydrate (HEPPSO), 3-[4-(2-hydroxyethyl)-1-piperazinyl]propanesulfonic acid (HEPPS), tricine, glycinamide, bicine or N-tris(hydroxymethyl)methyl-3-aminopropanesulfonic acid sodium (TAPS); glycine; diethanolamine (DEA); and combinations thereof.

Amino acid buffers include, but are not limited to, taurine, aspartic acid and its salts (e.g., potassium salts, etc), ε-aminocaproic acid, and combinations thereof.

The ophthalmic formulation comprises from 0.05 wt % to 1 wt % of one or more buffers. In some aspects, the ophthalmic formulation comprises 0.05 wt %, about 0.1 wt %, about 0.15 wt %, about 0.2 wt %, about 0.25 wt %, about 0.4 wt %, about 0.45 wt %, about 0.5 wt %, about 0.55 wt %, about 0.6 wt %, about 0.65 wt %, about 0.7 wt %, about 0.75 wt %, about 0.8 wt %, about 0.85 wt %, about 0.9 wt %, 0.95 wt %, or 1 wt % of one or more buffers. In some aspects, the ophthalmic formulation comprises about 0.3 wt % of one or more buffers. In some aspects, the ophthalmic formulation comprises about 0.35 wt % of one or more buffers.

In some aspects, the pH of the ophthalmic formulation is adjusted by a strong acid or base. Examples of strong acids and strong bases are well known in the art and include, without limitation, NaOH, KOH, HCl, and H₂SO₄. In some aspects, the strong acid or base is HCl or NaOH.

In some aspects, the ophthalmic formulation comprises a preservative. Non-limiting examples of preservatives include benzalkonium chloride, stabilized oxychloro complexes (Purite^{®}), phenylmercuric acetate, chlorobutanol, benzyl alcohol, parabens, EDTA and thimerosal.

In some aspects, the ophthalmic formulation does not contain a preservative.

In some aspects, the ophthalmic formulation does not contain etheylenediaminetetraacetic acid (EDTA).

In some aspects, the ophthalmic formulation comprises a permeation enhancer. Non-limiting examples of permeation enhancers include benzalkonium chloride, laurocapram (azone), bile acids and their alkali metal salts, including chenodeoxycholoc acid, cholic acid, taurocholic acid, taurodeoxycholic acid, tauroursodeoxycholic acid or ursodeoxycholic acid, glycocholate, n-dodecyl-β-D-maltoside, sucrose dodecanoate, octyl maltoside, decyl maltoside, tridecyl maltoside, tetradecyl maltoside, hexamethylene lauramide, hexamethylene octanamide, glycerol monolaurate, PGML (polyethylene glycol monolaurate), dimethyl sulfoxide, methylsulfonylmethane, sodium fusidate, saponins or any combination thereof. In some aspects, the ophthalmic formulation comprises benzalkonium chloride.

In some aspects, the ophthalmic formulation comprises from about 0.0025 wt % to about 0.005 wt %, from about 0.0025 wt % to about 0.0075 wt %, from about 0.0025 wt % to about 0.01 wt %, from about 0.0025 wt % to about 0.0125 wt %, from about 0.0025 wt % to about 0.02 wt %, from about 0.005 wt % to about 0.0075 wt %, from about 0.005 wt % to about 0.01 wt %, from about 0.005 wt % to about 0.0125 wt %, from about 0.005 wt % to about 0.02 wt %, from about 0.0075 wt % to about 0.01 wt %, from about 0.0075 wt % to about 0.02 wt %, from about 0.01 wt % to about 0.0125 wt %, from about 0.01 wt % to about 0.02 wt %, or from about 0.0125 wt % to about 0.02 wt % permeation enhancer. In some aspects, the ophthalmic formulation comprises from about 0.0075 wt % to about 0.0125 wt % permeation enhancer.

In some aspects, the ophthalmic formulation comprises about 0.0025 wt %, about 0.005 wt %, about 0.0075 wt %, about 0.0110 wt %, about 0.0115 wt %, about 0.0125 wt %, or about 0.02 wt % permeation enhancer. In some aspects, the ophthalmic formulation comprises about 0.01 wt % permeation enhancer.

In some aspects, the ophthalmic formulation comprises from about 0.0025 wt % to about 0.005 wt %, from about 0.0025 wt % to about 0.0075 wt %, from about 0.0025 wt % to about 0.01 wt %, from about 0.0025 wt % to about 0.0125 wt %, from about 0.0025 wt % to about 0.02 wt %, from about 0.005 wt % to about 0.0075 wt %, from about 0.005 wt % to about 0.01 wt %, from about 0.005 wt % to about 0.0125 wt %, from about 0.005 wt % to about 0.02 wt %, from about 0.0075 wt % to about 0.01 wt %, from about 0.0075 wt % to about 0.02 wt %, from about 0.01 wt % to about 0.0125 wt %, from about 0.01 wt % to about 0.02 wt %, or from about 0.0125 wt % to about 0.02 wt % benzalkonium chloride. In some aspects, the ophthalmic formulation comprises from about 0.0075 wt % to about 0.0125 wt % benzalkonium chloride.

In some aspects, the ophthalmic formulation comprises about 0.0025 wt %, about 0.005 wt %, about 0.0075 wt %, about 0.0110 wt %, about 0.0115 wt %, about 0.0125 wt %, or about 0.02 wt % benzalkonium chloride. In some aspects, the ophthalmic formulation comprises about 0.01 wt % benzalkonium chloride.

In some aspects, the ophthalmic formulation does not contain a permeation enhancer.

In some aspects, the ophthalmic formulation does not contain benzalkonium chloride.

In some aspects, the ophthalmic formulation comprises one or more stabilizers. Stabilizers include, but are not limited to, fatty acids, fatty alcohols, alcohols, long chain fatty acid esters, long chain ethers, hydrophilic derivatives of fatty acids, polyvinyl pyrrolidones, polyvinyl ethers, polyvinyl alcohols, hydrocarbons, hydrophobic polymers, moisture-absorbing polymers, and combinations thereof. In some aspects, amide analogues of stabilizers are also used. In some aspects, the chosen stabilizer changes the hydrophobicity of the formulation, improves the mixing of various components in the formulation, controls the moisture level in the formula, or controls the mobility of the phase.

In some aspects, the ophthalmic formulation comprises one or more stabilizers in sufficient amounts to inhibit the degradation of the active agents. Examples of such stabilizing agents, include, but are not limited to: glycerol, methionine, monothioglycerol, EDTA, ascorbic acid, polysorbate 80, polysorbate 20, arginine, heparin, dextran sulfate, cyclodextrins, pentosan polysulfate and other heparinoids, divalent cations such as magnesium and zinc, or combinations thereof.

In some aspects, the ophthalmic formulation does not contain etheylenediaminetetraacetic acid (EDTA).

In some aspects, the ophthalmic formulation comprises from about 0.01 wt % to about 5 wt % impurity A. In some aspects, the ophthalmic formulation comprises from about 0.01 wt % to about 0.1 wt %, from about 0.01 wt % to about 0.5 wt %, from about 0.01 wt % to about 1 wt %, from about 0.01 wt % to about 1.5 wt %, from about 0.01 wt % to about 2 wt %, from about 0.01 wt % to about 2.5 wt %, from about 0.01 wt % to about 3 wt %, from about 0.01 wt % to about 3.5 wt %, from about 0.01 wt % to about 4 wt %, from about 0.01 wt % to about 4.5 wt %, from about 0.1 wt % to about 0.5 wt %, from about 0.1 wt % to about 1 wt %, from about 0.1 wt % to about 1.5 wt %, from about 0.1 wt % to about 2 wt %, from about 0.1 wt % to about 2.5 wt %, from about 0.1 wt % to about 3 wt %, from about 0.1 wt % to about 3.5 wt %, from about 0.1 wt % to about 4 wt %, from about 0.1 wt % to about 4.5 wt %, from about 0.1 wt % to about 5 wt %, from about 0.5 wt % to about 1 wt %, from about 0.5 wt % to about 1.5 wt %, from about 0.5 wt % to about 2 wt %, from about 0.5 wt % to about 2.5 wt %, from about 0.5 wt % to about 3 wt %, from about 0.5 wt % to about 3.5 wt %, from about 0.5 wt % to about 4 wt %, from about 0.5 wt % to about 4.5 wt %, from about 0.5 wt % to about 5 wt %, from about 1 wt % to about 1.5 wt %, from about 1 wt % to about 2 wt %, from about 1 wt % to about 2.5 wt %, from about 1 wt % to about 3 wt %, from about 1 wt % to about 3.5 wt %, from about 1 wt % to about 4 wt %, from about 1 wt % to about 4.5 wt %, from about 1 wt % to about 5 wt %, from about 1.5 wt % to about 2 wt %, from about 1.5 wt % to about 2.5 wt %, from about 1.5 wt % to about 3 wt %, from about 1.5 wt % to about 3.5 wt %, from about 1.5 wt % to about 4 wt %, from about 1.5 wt % to about 4.5 wt %, from about 1.5 wt % to about 5 wt %, from about 2 wt % to about 2.5 wt %, from about 2 wt % to about 3 wt %, from about 2 wt % to about 3.5 wt %, from about 2 wt % to about 4 wt %, from about 2 wt % to about 4.5 wt %, from about 2 wt % to about 5 wt %, from about 2.5 wt % to about 3 wt %, from about 2.5 wt % to about 3.5 wt %, from about 2.5 wt % to about 4 wt %, from about 2.5 wt % to about 4.5 wt %, from about 2.5 wt % to about 5 wt %, from about 3 wt % to about 3.5 wt %, from about 3 wt % to about 4 wt %, from about 3 wt % to about 4.5 wt %, from about 3 wt % to about 5 wt %, from about 3.5 wt % to about 4 wt %, from about 3.5 wt % to about 4.5 wt %, from about 3.5 wt % to about 5 wt %, from about 4 wt % to about 4.5 wt %, from about 4 wt % to about 5 wt %, or from about 4.5 wt % to about 5 wt % impurity A.

In some aspects, the ophthalmic formulation comprises about 5 wt % impurity A. In some aspects, the ophthalmic formulation comprises about 4.5 wt %, about 4 wt %, about 3.5 wt %, about 3 wt %, about 2.5 wt %, about 2 wt %, about 1.5 wt %, about 1 wt %, about 0.5 wt %, about 0.1 wt %, or about 0.01 wt % impurity A.

In some aspects, the ophthalmic formulation does not contain impurity A.

In some aspects, the ophthalmic formulation comprises less than 5 wt % impurity A after storage for about 5 months. In some aspects, the ophthalmic formulation comprises less than 4.5 wt %, less than 4 wt %, less than 3.5 wt %, less than 3 wt %, less than 2.5 wt %, less than 2 wt %, less than 1.5 wt %, less than 1 wt %, less than 0.5 wt %, less than 0.1 wt %, or less than 0.01 wt % impurity A after storage for about 5 months.

In some aspects, the ophthalmic formulation comprises from about 0.01 wt % to about 5 wt % impurity B. In some aspects, the ophthalmic formulation comprises from about 0.01 wt % to about 0.1 wt %, from about 0.01 wt % to about 0.5 wt %, from about 0.01 wt % to about 1 wt %, from about 0.01 wt % to about 1.5 wt %, from about 0.01 wt % to about 2 wt %, from about 0.01 wt % to about 2.5 wt %, from about 0.01 wt % to about 3 wt %, from about 0.01 wt % to about 3.5 wt %, from about 0.01 wt % to about 4 wt %, from about 0.01 wt % to about 4.5 wt %, from about 0.1 wt % to about 0.5 wt %, from about 0.1 wt % to about 1 wt %, from about 0.1 wt % to about 1.5 wt %, from about 0.1 wt % to about 2 wt %, from about 0.1 wt % to about 2.5 wt %, from about 0.1 wt % to about 3 wt %, from about 0.1 wt % to about 3.5 wt %, from about 0.1 wt % to about 4 wt %, from about 0.1 wt % to about 4.5 wt %, from about 0.1 wt % to about 5 wt %, from about 0.5 wt % to about 1 wt %, from about 0.5 wt % to about 1.5 wt %, from about 0.5 wt % to about 2 wt %, from about 0.5 wt % to about 2.5 wt %, from about 0.5 wt % to about 3 wt %, from about 0.5 wt % to about 3.5 wt %, from about 0.5 wt % to about 4 wt %, from about 0.5 wt % to about 4.5 wt %, from about 0.5 wt % to about 5 wt %, from about 1 wt % to about 1.5 wt %, from about 1 wt % to about 2 wt %, from about 1 wt % to about 2.5 wt %, from about 1 wt % to about 3 wt %, from about 1 wt % to about 3.5 wt %, from about 1 wt % to about 4 wt %, from about 1 wt % to about 4.5 wt %, from about 1 wt % to about 5 wt %, from about 1.5 wt % to about 2 wt %, from about 1.5 wt % to about 2.5 wt %, from about 1.5 wt % to about 3 wt %, from about 1.5 wt % to about 3.5 wt %, from about 1.5 wt % to about 4 wt %, from about 1.5 wt % to about 4.5 wt %, from about 1.5 wt % to about 5 wt %, from about 2 wt % to about 2.5 wt %, from about 2 wt % to about 3 wt %, from about 2 wt % to about 3.5 wt %, from about 2 wt % to about 4 wt %, from about 2 wt % to about 4.5 wt %, from about 2 wt % to about 5 wt %, from about 2.5 wt % to about 3 wt %, from about 2.5 wt % to about 3.5 wt %, from about 2.5 wt % to about 4 wt %, from about 2.5 wt % to about 4.5 wt %, from about 2.5 wt % to about 5 wt %, from about 3 wt % to about 3.5 wt %, from about 3 wt % to about 4 wt %, from about 3 wt % to about 4.5 wt %, from about 3 wt % to about 5 wt %, from about 3.5 wt % to about 4 wt %, from about 3.5 wt % to about 4.5 wt %, from about 3.5 wt % to about 5 wt %, from about 4 wt % to about 4.5 wt %, from about 4 wt % to about 5 wt %, or from about 4.5 wt % to about 5 wt % impurity B.

In some aspects, the ophthalmic formulation comprises about 5 wt % impurity B. In some aspects, the ophthalmic formulation comprises about 4.5 wt %, about 4 wt %, about 3.5 wt %, about 3 wt %, about 2.5 wt %, about 2 wt %, about 1.5 wt %, about 1 wt %, about 0.5 wt %, about 0.1 wt %, or about 0.01 wt % impurity B.

In some aspects, the ophthalmic formulation does not contain impurity B.

In some aspects, the ophthalmic formulation comprises less than 5 wt % impurity B after storage for about 5 months. In some aspects, the ophthalmic formulation comprises less than 4.5 wt %, less than 4 wt %, less than 3.5 wt %, less than 3 wt %, less than 2.5 wt %, less than 2 wt %, less than 1.5 wt %, less than 1 wt %, less than 0.5 wt %, less than 0.1 wt %, or less than 0.01 wt % impurity B after storage for about 5 months.

In some aspects, the ophthalmic formulation is stored at 40 °C and NMT 25% relative humidity. In some aspects, the ophthalmic formulation is stored at 25 °C and 40% relative humidity. In some aspects, the ophthalmic formulation is stored for about 1 day, about 7 days, about 21 days, about 1 month, about 2 months, about 3 months, about 4 months, about 5 months, about 6 months, or about 12 months.

In some aspects, the ophthalmic formulation comprises 2.75 wt % carbachol, 0.1 wt % brimonidine, 0.2 wt % HPMC, and from 0.05 wt % to 1 wt % of one or more buffers, wherein the pH is 7.4, and wherein the formulation comprises less than 5 wt % impurity A after storage for 5 months.

In some aspects, the ophthalmic formulation comprises 2.75 wt % carbachol, 0.1 wt % brimonidine, 0.2 wt % HPMC, and from 0.05 wt % to 1 wt % of one or more buffers, wherein the pH is 7.4, and wherein the formulation comprises less than 2 wt % impurity B after storage for 5 months.

In some aspects, the ophthalmic formulation has a total impurity concentration of less than 1 wt %. In some aspects, the ophthalmic formulation has a total impurity concentration of less than 0.5 wt %, less than 0.1 wt %, less than 0.05 wt %, less than 0.01 wt %, or less than 0.005 wt %.

In some aspects, the solubility of the components of the ophthalmic formulation may be enhanced by a surfactant or other appropriate co-solvent in the composition. Such co-solvents include polysorbate 20, 60, and 80, Pluronic F68, F-84 and P-103, cyclodextrin, or other agents known to those skilled in the art. In some aspects, the concentration of the co-solvent is from 0.01 wt % to about 2 wt %.

In some aspects, the ophthalmic formulation comprises one or more polyols. As used herein, the term "polyol" includes any compound having at least one hydroxyl group on each of two adjacent carbon atoms that are not in trans configuration relative to each other. The polyol can be linear or cyclic, substituted or unsubstituted, or mixtures thereof, so long as the resultant complex is water soluble and pharmaceutically acceptable. Examples of such compounds include: sugars, sugar alcohols, sugar acids and uronic acids. Preferred polyols are sugars, sugar alcohols and sugar acids, including, but not limited to: mannitol, glycerin, xylitol, sorbitol and propylene glycol. It is contemplated that the polyol may be comprised of two or more different polyols.

In some aspects, the ophthalmic formulation comprises one or more anti-aggregation additives. Anti-aggregation additives enhance stability of the ophthalmic formulation by reducing the rate of protein aggregation. Anti-aggregation additives include, but are not limited to, urea, guanidinium chloride, simple amino acids such as glycine or arginine, sugars, polyalcohols, polysorbates, polymers such as polyethylene glycol and dextrans, alkyl saccharides, such as alkyl glycoside, and surfactants.

In some aspects, the ophthalmic formulation comprises one or more antioxidants. Antioxidants include, but are not limited to, ascorbic acid, methionine, sodium thiosulfate, sodium metabisulfite, and combinations thereof. Metal chelating agents, thiol-containing compounds, and other general stabilizing agents may be acceptable antioxidants.

In some aspects, the ophthalmic formulation comprises one or more osmolality agents. Osmolality agents include, but are not limited to, salts, particularly sodium chloride or potassium chloride, organic compounds such as propylene glycol, mannitol, sorbitol, dextrose, and glycerin.

In some aspects, the ophthalmic formulation has an osmolality of from about 260 to about 365 mOsm/kg. In some aspects, the ophthalmic formulation has an osmolality of from about 285 mOsm/kg to about 295 mOsm/kg, from about 285 mOsm/kg to about 305 mOsm/kg, from about 285 mOsm/kg to about 315 mOsm/kg, from about 285 mOsm/kg to about 325 mOsm/kg, from about 285 mOsm/kg to about 335 mOsm/kg, from about 285 mOsm/kg to about 345 mOsm/kg, from about 285 mOsm/kg to about 355 mOsm/kg, from about 285 mOsm/kg to about 365 mOsm/kg, from about 295 mOsm/kg to about 305 mOsm/kg, from about 295 mOsm/kg to about 315 mOsm/kg, from about 295 mOsm/kg to about 325 mOsm/kg, from about 295 mOsm/kg to about 335 mOsm/kg, from about 295 mOsm/kg to about 345 mOsm/kg, from about 295 mOsm/kg to about 355 mOsm/kg, from about 295 mOsm/kg to about 365 mOsm/kg, from about 305 mOsm/kg to about 315 mOsm/kg, from about 305 mOsm/kg to about 325 mOsm/kg, from about 305 mOsm/kg to about 335 mOsm/kg, from about 305 mOsm/kg to about 345 mOsm/kg, from about 305 mOsm/kg to about 355 mOsm/kg, from about 305 mOsm/kg to about 365 mOsm/kg, from about 315 mOsm/kg to about 325 mOsm/kg, from about 315 mOsm/kg to about 335 mOsm/kg, from about 315 mOsm/kg to about 345 mOsm/kg, from about 315 mOsm/kg to about 355 mOsm/kg, from about 315 mOsm/kg to about 365 mOsm/kg, from about 325 mOsm/kg to about 335 mOsm/kg, from about 325 mOsm/kg to about 345 mOsm/kg, from about 325 mOsm/kg to about 355 mOsm/kg, from about 325 mOsm/kg to about 365 mOsm/kg, from about 335 mOsm/kg to about 345 mOsm/kg, from about 335 mOsm/kg to about 355 mOsm/kg, from about 335 mOsm/kg to about 365 mOsm/kg, from about 345 mOsm/kg to about 355 mOsm/kg, from about 345 mOsm/kg to about 365 mOsm/kg, from about 355 mOsm/kg to about 365 mOsm/kg, from about 260 mOsm/kg to about 265 mOsm/kg, from about 260 mOsm/kg to about 275 mOsm/kg, from about 260 mOsm/kg to about 285 mOsm/kg, from about 260 mOsm/kg to about 295 mOsm/kg, from about 260 mOsm/kg to about 305 mOsm/kg, from about 260 mOsm/kg to about 315 mOsm/kg, from about 260 mOsm/kg to about 325 mOsm/kg, from about 260 mOsm/kg to about 335 mOsm/kg, from about 260 mOsm/kg to about 345 mOsm/kg, from about 260 mOsm/kg to about 355 mOsm/kg, from about 260 mOsm/kg to about 365 mOsm/kg, from about 265 mOsm/kg to about 275 mOsm/kg, from about 265 mOsm/kg to about 285 mOsm/kg, from about 265 mOsm/kg to about 295 mOsm/kg, from about 265 mOsm/kg to about 305 mOsm/kg, from about 265 mOsm/kg to about 315 mOsm/kg, from about 265 mOsm/kg to about 325 mOsm/kg, from about 265 mOsm/kg to about 335 mOsm/kg, from about 265 mOsm/kg to about 345 mOsm/kg, from about 265 mOsm/kg to about 355 mOsm/kg, from about 265 mOsm/kg to about 365 mOsm/kg, from about 275 mOsm/kg to about 285 mOsm/kg, from about 275 mOsm/kg to about 295 mOsm/kg, from about 275 mOsm/kg to about 305 mOsm/kg, from about 275 mOsm/kg to about 315 mOsm/kg, from about 275 mOsm/kg to about 325 mOsm/kg, from about 275 mOsm/kg to about 335 mOsm/kg, from about 275 mOsm/kg to about 345 mOsm/kg, from about 275 mOsm/kg to about 355 mOsm/kg, from about 275 mOsm/kg to about 365 mOsm/kg.

In some aspects, the ophthalmic formulation has an osmolality of about 260 mOsm/kg, about 265 mOsm/kg, about 275 mOsm/kg, about 285 mOsm/kg, about 295 mOsm/kg, about 305 mOsm/kg, about 315 mOsm/kg, about 325 mOsm/kg, about 335 mOsm/kg, about 345 mOsm/kg, about 355 mOsm/kg, about 365 mOsm/kg, about 370 mOsm/kg, or about 375 mOsm/kg.

In some aspects, the ophthalmic formulation is isotonic. In some aspects, the ophthalmic formulation is hypotonic. In some aspects, the ophthalmic formulation is hypertonic.

In some aspects, the ophthalmic formulation can include a variety of additional ingredients. Such ingredients include, without limitation, additional therapeutic agents, additional or alternative antimicrobial agents, suspension agents, surfactants, additional or alternative tonicity agents, additional or alternative buffering agents, anti-oxidants, additional or alternative viscosity-modifying agents, chelating agents, or any combinations thereof.

### Methods of Treatment

The present disclosure provides methods of treating ocular conditions in a subject in need thereof, comprising administering a topical ophthalmic composition comprising carbachol and brimonidine. As used herein, the term "ocular condition" may refer to any condition, disease, or impairment, which affects or involves the eye or one of the parts or regions of the eye, and includes optical issues causing refractive errors in the eye. Ocular conditions include, but are not limited to presbyopia, myopia, progressive myopia, pathologic myopia, amblyopia, cycloplegia, mydriasis, allergic conjunctivitis, conjunctival hyperemia, red eye, glaucoma, ocular hypertension, night vision symptoms post refractive surgery (e.g ., glare, halos or starbursts around lights), accommodative esotropia, glaucoma, ocular hypertension, accommodative insufficiency, hyperopia, anisocoria, astigmatism, amblyopia, Adie's tonic pupil, or other causes of parasympathetic denervation, complications arising after refractive surgery, such as decentered ablations following LASIK or PRK, LASIK undercorrections, LASIK overcorrections, corneal scars, hazing, and refractive errors. In some aspects, the ocular condition is presbyopia.

"Presbyopia" is farsightedness often caused by loss of elasticity of the lens of the eye, occurring typically m middle and old age. Presbyopia is a condition associated with the aging of the eye that results in progressively worsening ability to focus clearly (particularly at close distance). Symptoms include difficulty reading small print, having to hold reading material farther away, headaches, and eyestrain. Most people begin to notice the effects of presbyopia sometime after age 40, when they start having trouble seeing small print clearly - including text messages on their phone. Application of cholinergic agonists (miotic agents) in these subjects is beneficial as the miosis resulting from sphincter muscle contraction creates a "pin-hole effect" that may potentially improve the near and intermediate vision by increasing the depth of field. These cholinergic agonists can thus be used for the treatment of presbyopia, although most effective dosing frequency and dose concentrations have not been defined.

A pinhole camera cuts down on the amount of light going in. Since the more light that is let in requires more focus, the user hardly needs to focus with pinhole cameras. Using a pinhole removes the optical periphery. The treatment methods and compositions described herein use drugs to get the pinhole effect, thus increasing depth of focus dramatically. There are two types of muscles in the eyes: constrictor muscles and dilator muscles. By acting on both of these types of muscles, the unique combination of drugs described herein are able to accomplish a pinhole effect, correcting refractive errors.

A pharmacologic pinhole effect is induced in at least the non-dominant eyes of any patients with refractive errors. In some aspects, the treatment may be administered in both eyes. In some aspects, the treatment is only administered in the non-dominant eye of emmetropic presbyopes and myopic presbyopes and in both eyes of hyperopic presbyopes and hyperopes. For pure myopes, the pinhole effect may be induced in either the non-dominant eye or both eyes of the myopes.

More specifically, carbachol causes the pupil to become small (constriction), and brimonidine acts as a paralyzer (preventing dilation). Brimonidine prevents pupillary dilation, that occurs at night, to minimize optical aberrations causing halos and glare in some patients after refractive surgery, as well as to treat glaucoma.

For some of the refractive errors including, but not limited to, presbyopia, the formulations are placed in only one eye, to decrease the likelihood of dimness from the treatment. For other refractive errors including, but not limited to, hyperopia, the drops are preferably placed in both eyes during treatment, but may alternatively be placed only in a single eye. In patients with myopic vision, pseudophakes, or astigmatism, the formulations may be placed in a single eye or in both eyes.

For some refractive errors, it may also be beneficial to administer the pharmaceutical preparations described herein to only a single eye of a patient. In some aspects, blurring of distance vision (a result of accommodative focus) and dimness of vision (a result of pupil constriction) may occur when the compositions are administered to both eyes of a patient. When applied to only a single eye, the benefits of improvement in presbyopia are obtained with diminished or complete relief of blurring and dimness. It was originally believed that a patient's brain compensates between the treated and untreated eyes thereby reducing the undesired effects. Therefore, the combination of a constricted pupil with its increased depth of field in the treated eye and normal distance vision and brightness in the untreated eye will cause the brain to ignore any monocular blur at distance or near vision when only one eye was treated. However, when the pharmaceutical preparation is applied to both eyes, distance visual acuity is preserved, even though parasympathomimetic drugs, or pharmaceutically acceptable salts thereof, such as pilocarpine and carbachol alone cause a myopic shift, inducing near sightedness at the expense of distance vision while providing increased depth of focus. The addition of an alpha-2 agonist such as brimonidine or its pharmaceutically acceptable salt prevents the myopic shift and preserves distance visual acuity when applied to both eyes.

Presbyopia may be diagnosed using one or more types of ocular examination procedures, where such examinations may include testing of one or more of visual acuity, e.g., by use of a Snellen chart, Jaeger chart, Rosenbaum chart or ETDRS Near Chart, refraction, binocular vision and accommodation, plus lens to clear near vision, balanced range of accommodation, amplitude of accommodation, crossed cylinder test, accommodative convergence/accommodation, heterophoria and vergence, and vertical imbalance.

The present disclosure also provides methods of ameliorating or reducing presbyopia in a subject comprising administering an ophthalmic formulation described herein.

When used in the treatment of presbyopia, the methods described herein may result in the improvement of one or more characteristics of presbyopia. In some aspects, administration to a subject of an ophthalmic formulation described herein may result in a reduction in pupil diameter as compared with reduction in pupil diameter observed prior to administration of the ophthalmic formulation. The magnitude of the reduction may vary. In some aspects, the reduction is from about 0.25 mm to about 10 mm, from about 1 mm to about 9 mm, or from about 2 mm to about 8 mm. In some aspects, the reduction is from about 0.25 mm to about 3.0 mm, from about 0.5 mm to about 1.5 mm, or from about 0.5 mm to about 1.0 mm. In some aspects, the methods may result in an improvement in uncorrected near visual acuity or other measures of visual function including uncorrected intermediate or distance vision, contrast sensitivity or depth of focus, amongst other measures. Where visual acuity is measured using a chart, such as a Jaeger or Rosenbaum near card, Snellen card or ETDRS Near Chart, the methods described herein may result in an improvement of one or more lines on the chart, such as 1 to 8 lines or 2 to 6 lines. In some aspects, the magnitude of improvement with respect to a letter that can be read at about 20 feet is about 5 feet or more, about 10 feet or more, about 15 feet or more (1 foot = 0.3048 m). In some aspects, the magnitude improvement with respect to a letter that can be read at about 20 feet is from about 5 feet to about 60 feet, from about 5 feet to about 30 feet, or from about 10 feet to about 25 feet. In some aspects, visual acuity improves from about 20/40 to about 20/25 or from about 20/40 to about 20/20.

In some aspects, the amelioration or reduction of presbyopia is effective for at least 8 hours. In some aspects, the amelioration or reduction of presbyopia is effective for at least 0.5 hours, at least 1 hour, at least 1.5 hours, at least 2 hours, at least 2.5 hours, at least 3 hours, at least 3.5 hours, at least 4 hours, at least 4.5 hours, at least 5 hours, at least 5.5 hours, at least 6 hours, at least 6.5 hours, at least 7 hours, at least 7.5 hours, at least 8.5 hours, at least 9 hours, at least 9.5 hours, at least 10 hours, at least 12 hours, at least 16 hours, at least 20 hours, or at least 24 hours.

In some aspects, administration of the ophthalmic formulation reduces periorbital pain in the subject.

The present disclosure also provides methods of ameliorating or reducing at least one refractive error of subject with hyperopia, relaxing a ciliary muscle of a subject stimulated by sympathetic innervation to reduce at least one of headache, browache and periorbital pain, preventing a parasympathomimetic induced myopic shift in a patient with presbyopia receiving parasympathomimetic drugs or pharmaceutically acceptable salts thereof, ameliorating or reducing at least one refractive error of a pseudophakic patient selected from the group consisting of myopia, hyperopia, and astigmatism, treating at least one refractive error in a patient that has had ocular surgery, and creating multifocality in a pseudophakic patient, comprising administering an ophthalmic formulation described herein.

In some aspects, administering the ophthalmic formulation to a subject with hyperopia results in vision improvement of at least 20% relative to no treatment. In some aspects, administering the ophthalmic formulation to a subject with hyperopia results in vision improvement of at least 5%, at least 10%, at least 15%, at least 25%, at least 30%, at least 35%, at least 40%, at least 45%, at least 50%, at least 60%, at least 70%, at least 80%, at least 90%, or 100% relative to no treatment.

In some aspects, administration of the ophthalmic formulation results in an increase in the depth of focus in the vision of the subject.

In some aspects, administration of the ophthalmic formulation preserves visual acuity in the vision of the subject.

The ophthalmic preparation may be administered to a subject suffering from myopia, hyperopia, astigmatism, presbyopia or other optical errors as often as needed to cause miosis sufficient to temporarily treat, ameliorate, or reduce these optical errors as well as temporarily create multifocality. These refractive errors all benefit from these drugs to a clinically and practically usable degree which enable patients who needed glasses full time to totally do without them. Thus, the present disclosure further provides methods for temporarily treating, ameliorating, or reducing these optical errors by inducing miosis as well as temporarily creating multifocality.

"Optical errors", or "refractive errors", as defined herein, also known as ammetropia (vision abnormalities), are vision defects or optical imperfections that prevent the eye from properly focusing light, causing blurred vision. The primary refractive errors are myopia (nearsightedness), hyperopia (farsightedness, blurred vision), presbyopia (when the lens in the eye loses flexibility), pseudophakia (a near vision defect created by the implantation of an artificial intraocular lens) and astigmatism (including regular astigmatism, irregular astigmatism and high degrees of regular astigmatism). Some refractive errors occur after cataract surgery or laser surgery.

The terms "ameliorate, ameliorating, and amelioration," as used herein, are intended to refer to a decrease in the severity of the refractive error. The amelioration may be complete, e.g., the total absence of one or more refractive errors. The amelioration may also be partial, such that the amount of the refractive error is less than that which would have been present without the treatment. For example, the extent of the refractive errors using the methods of the present invention may be at least 10%, at least 20%, at least 30%, at least 40%, at least 50%, at least 60%, at least 70%, at least 80%, at least 90%, or at least 100% less than the amount of the refractive errors that would have been present without using these methods.

Methods described herein ameliorate refractive errors, including, but not limited to, myopia, hyperopia, astigmatism, presbyopia, pseudophakes (replacing a natural lens with an artificial intraocular lens, for example after cataract surgery), and distortions after laser surgery by administering to at least one eye of a patient a therapeutically effective amount of an ophthalmic preparation comprising one or more parasympathomimetic drugs, or pharmaceutically acceptable salts thereof, and one or more alpha agonists or antagonists, or pharmaceutically acceptable salts thereof.

The present disclosure further provides methods of improving at least one vision parameter in a subject in need thereof, comprising administering to the subject an ophthalmic formulation described herein. As used herein, the term "vision parameter" refers to any characteristic in a subject's vision that may be measured and is susceptible to being improved by the ophthalmic formulations and methods described herein. Vision parameters include, but are not limited to, near vision acuity, intermediate visual acuity, distance visual acuity, night vision, day vision, optical aberrations (e.g., glare, light scattering), and uncorrected refractive errors. Additional examples of vision parameters include, without limitation, night time glare, post-LASIK "star burst" glare, visual "halos" seen around light sources, and accommodative insufficiency.

"Improving vision parameter" includes, but is not limited to, near, intermediate, and/or distance visual acuity, and may for example be reflected in the increase of number of letters correctly read at any time point post dosing, the increase in the average letter change, or 2-line or 3-line improvement, all from baseline (i.e., from pre-treatment).

Night vision improvement may be reflected in visual improvement for subjects in dim or dark lighting (e.g., under mesopic or scotopic conditions). Day vision improvement may be reflected in visual improvement for subjects in bright lighting as found during daylight hours or in sunshine (e.g., under photopic conditions). Vision improvement using the methods described herein may also be achieved in combination with or when using other visual aids and devices (e.g., those used for treating myopia or presbyopia), including but not limited to reading glasses, lens modifying medications, and surgical myopic options.

In some aspects, methods of treatment using the ophthalmic formulations described herein result in an at least 2-line improvement from baseline under the condition of photopic, high contrast uncorrected near visual acuity (UNVA). As used herein, the term "photopic" vision is the vision of the eye under well-lit conditions (luminance level 10 to 10⁸ cd/m²). In humans and other animals, photopic vision allows color perception, mediated by cone cells, and a significantly higher visual acuity and temporal resolution than available with scotopic vision (the vision of the eye under low-light conditions; luminance level 10⁻³ to 10⁻⁶ cd/m²). As used herein, the term "uncorrected near visual acuity" (UNVA) refers to a subject's ability, without any vision aid (such as eyeglasses or contact lenses), to see the details of objects within arm's distance from the body (e.g., at 33-41 cm away from the eye).

In some aspects, methods of treatment using the ophthalmic formulation described herein result in an at least 3-line improvement from baseline under the condition of photopic, high contrast UNVA. In other aspects, methods described herein result in an increase in the average letter change from baseline under the condition of photopic, high contrast UNVA.

The term "improvement from baseline" refers to the increase from pre-treatment in the number of letters correctly read at certain post treatment time point. As used herein, the term "2-line improvement from baseline" or "3 -line improvement from baseline" or similar improvement from baseline refers to a subject's ability to read 2 or 3 more lines of letters on a standard chart (e.g., Snellen, ETDRS, Logarithmic Visual Acuity Chart, etc.) after treatment with a topical ophthalmic composition of the invention when comparing to the number of lines readable before treatment.

In some aspects, methods of treatment using the ophthalmic formulations described herein result in an at least 2-line improvement from baseline under the condition of mesopic, high contrast UNVA. As used herein, the term "mesopic" vision refers to a combination of photopic vision and scotopic vision in low but not quite dark lighting situations. Mesopic light levels range from luminances of approximately 0.001 to 3 cd/m². Most night-time outdoor and traffic lighting scenarios are in the mesopic range. The human eye uses scotopic vision under low-light conditions and mesopic vision in intermediate conditions. Humans see differently at different light levels. This is because under high light levels typical during the day (photopic vision), the eye uses cones to process light. Under very low light levels, corresponding to moonless nights without electric lighting (scotopic vision), the eye uses rods to process light. At many night-time levels, a combination of both cones and rods supports vision. Photopic vision facilitates excellent color discrimination ability, whereas colors are indiscriminable under scotopic vision. Mesopic vision falls between these two extremes. In most night time environments, there is enough ambient light at night to prevent true scotopic vision.

In some aspects, methods of treatment using the topical ophthalmic compositions described herein result in an at least 3-line improvement from baseline under the condition of mesopic, high contrast UNVA. In other aspects, methods described herein result in an increase in the average letter change from baseline under the condition of mesopic, high contrast UNVA.

In some aspects, methods of treatment using the ophthalmic formulations described herein result in an at least 2-line improvement from baseline under the condition of photopic, high contrast uncorrected distance visual acuity (UDVA). As used herein, the term "uncorrected distance visual acuity" (UDVA) refers to a subject's ability, without any vision aid (such as eyeglasses or contact lenses), to see the details of objects beyond arm's distance from the body (e.g., greater than 4 meters away from the eye).

In some aspects, methods of treatment using the ophthalmic formulations described herein result in an at least 3-line improvement from baseline under the condition of photopic, high contrast UDVA. In other embodiments, methods described herein result in an increase in the average letter change from baseline under the condition of photopic, high contrast UDVA.

In some aspects, methods of treatment using the ophthalmic formulations described herein result in an at least 2-line improvement from baseline under the condition of mesopic, high contrast distance-corrected near visual acuity (DCNVA). As used herein, the term "distance corrected near visual acuity" (DCNVA) refers to a subject's ability to see the details of objects within arm's distance from the body (e.g., at 33-41 cm away from the eye), with the use of vision aids such as eyeglasses or contact lenses that correct for distance vision issues.

In some aspects, methods of treatment using the ophthalmic formulations described herein result in an at least 3-line improvement from baseline under the condition of mesopic, high contrast DCNVA. In other aspects, methods described herein result in an increase in the average letter change from baseline under the condition of mesopic, high contrast DCNVA. In yet other aspects, methods described herein result in an at least 3-line improvement from baseline under the condition of photopic, high contrast DCNVA. In additional aspects, methods described herein result in an at least 2-line improvement from baseline under the condition of photopic, high contrast DCNVA. In further aspects, methods described herein result in an increase in the average letter change from baseline under the condition of photopic, high contrast DCNVA.

In certain aspects, methods of treatment using the ophthalmic formulations described herein result in an at least 2-line improvement from baseline under the condition of mesopic, high contrast distance-corrected intermediate visual acuity (DCIVA). As used herein, the term "distance-corrected intermediate visual acuity" (DCIVA) may be used to refer to a subject's ability to see the details of objects at intermediate distances with the use of vision aids such as eyeglasses or contact lenses that correct for distance vision issues.

In some aspects, methods of treatment using the ophthalmic formulations described herein result in an at least 3-line improvement from baseline under the condition of mesopic, high contrast DCIVA. In other aspects, methods described herein result in an increase in the average letter change from baseline under the condition of mesopic, high contrast DCIVA. In yet other aspects, methods described herein result in an at least 2-line improvement from baseline under the condition of photopic, high contrast DCIVA. In additional aspects, methods described herein result in an at least 3-line improvement from baseline under the condition of photopic, high contrast DCIVA. In further aspects, methods described herein result in an increase in the average letter change from baseline under the condition of photopic, high contrast DCIVA.

### Processes for Preparing Ophthalmic Formulations

The present disclosure also provides processes (not claimed) for preparing ophthalmic formulations comprising carbachol and brimonidine.

In some aspects, an ophthalmic formulation comprising from about 2 wt % to about 4 wt % carbachol, or a pharmaceutically acceptable salt thereof, from about 0.05 wt % to about 0.2 wt % brimonidine, or a pharmaceutically acceptable salt thereof, from about 0.05 wt % to about 1 wt % of one or more viscosity agents, and from about 0.05 wt % to about 1 wt % of one or more buffers, wherein the pH of the formulation is from about 7 to about 7.6, is prepared by a process comprising adding carbachol, or a pharmaceutically acceptable salt thereof, brimonidine, or a pharmaceutically acceptable salt thereof, and one or more viscosity agents to water and mixing to provide the formulation.

In some aspects, the buffer is a phosphate buffer. In some aspects, the phosphate buffer comprises sodium phosphate monobasic monohydrate and sodium phosphate dibasic heptahydrate.

In some aspects, the one or more viscosity agents is HPMC.

In some aspects, the process comprises adding benzalkonium chloride.

In some aspects, the process comprises adding sodium chloride.

In some aspects, the process comprises adding hydrochloric acid.

In some aspects, the process comprises adding sodium hydroxide.

In some aspects, the ophthalmic formulation is aseptically filled into vials.

In some aspects, each vial is filled with from about 0.01 g to about 0.1 g, from about 0.05 g to about 0.15 g, from about 0.2 g to about 0.4 g, from about 0.3 g to about 0.5 g, from about 1 g to about 2 g, from about 1.5 g to about 2.5 g, from about 2.5 g to about 3.5 g, from about 3 g to about 4 g, or from about 3.5 g to about 5 g of the ophthalmic formulation.

In some aspects, each vial is filled with from about 0.1 g to about 0.3 g of the ophthalmic formulation.

In some aspects, each vial is filled with from about 2 g to about 2.7 g of the ophthalmic formulation.

The following examples are illustrative and do not limit the scope of the claimed aspects.

### EXAMPLES

### Example 1

### Exemplary Carbachol/Brimonidine Formulations

Table 1 lists exemplary ophthalmic formulations comprising carbachol and brimonidine that were prepared for further testing. Opthalmic formulations which do not fall within the scope of the claims are described for reference only.

**Table 1: Carbachol/Brimonidine Formulations**

| | | Formula 1 | Formula 2 | Formula 3 | Formula 4 | Formula 5 | Formula 6 | Formula 7 | Formula 8 | Formula 9 | Formula 10 |
|---|---|---|---|---|---|---|---|---|---|---|---|
| **Component** | **Grade** | **Conc (wt. %)** | **Conc (wt. %)** | **Conc (wt. %)** | **Conc (wt. %)** | **Conc (wt. %)** | **Conc (wt. %)** | **Conc (wt. %)** | **Conc (wt. %)** | **Conc (wt. %)** | **Conc (wt. %)** |
| Carbachol | USP | 3.0% | 2.75% | 2.75% | 2.75% | 2.75% | 2.75% | NA | 2.75% | 2.25% | 2.75% |
| Brimonidine Tartrate | Non-Comp endial | 0.2% | 0.1% | 0.1% | 0.1% | NA | 0.1% | 0.1% | NA | NA | NA |
| CMC | USP/P h Eur | 1.0% | NA | NA | NA | NA | NA | NA | NA | NA | NA |
| Hydroxypropyl methyl Cellulose (HPMC) | USP/P h Eur | NA | 0.2% | 0.2% | 0.2% | .2% | 0.2% | 0.2% | 0.2% | 0.2% | .2% |
| Benzalkonium Chloride | NF/Ph Eur | 100 ppm | 100 ppm | NA | NA | 100 ppm | 100 ppm | NA | NA | NA | NA |
| Edetate Disodium | USP | 0.05% | 0.05% | 0.05% | NA | NA | NA | 0.05% | 0.05% | NA | NA |

### Example 2

### Additional Exemplary Carbachol/Brimonidine Formulations

Table 2 lists additional exemplary ophthalmic formulations comprising carbachol and brimonidine were prepared for further testing. Opthalmic formulations which do not fall within the scope of the claims are described for reference only.

**Table 2: Additional Carbachol/Brimonidine Formulations**

| **Formulation#** | | **Formula 11** | **Formula 12** | **Formula 13** | **Formula 14** | **Formula 15** | **Formula 16** | **Formula 17** | **Formula 18** | **Formula 19** | **Formula 20** | **Formula 21** |
|---|---|---|---|---|---|---|---|---|---|---|---|---|
| **Component** | **Grade** | **Conc (wt. %)** | **Conc (wt. %)** | **Conc (wt. %)** | **Conc (wt. %)** | **Conc (wt. %)** | **Conc (wt. %)** | **Conc (wt. %)** | **Conc (wt. %)** | **Conc (wt. %)** | **Conc (wt. %)** | **Conc (wt. %)** |
| Carbachol | USP | 3% | 2.75% | 3% | 2.75% | 2.75% | 2.75% | 2.75% | 2.75% | 2.75% | 2.75% | 2.75% |
| Brimonidine Tartrate | Non-Compendi al | 0.2% | 0.1% | 0.2% | 0.1% | 0.1% | 0.1% | 0.1% | 0.1% | 0.1% | 0.1% | 0.1% |
| Sodium Carboxymethyl Cellulose (NaCMC, Grade 7M8SFPH) | USP/Ph Eur | 1.0% | 1.0% | 1.0% | - | - | - | - | - | 1.0% | - | - |
| Hydroxypropyl methyl Cellulose (HPMC, Grade Methocel E50 Premium LY) | USP/Ph Eur | - | - | - | 0.2% | 0.2% | 1.0% | 1.0% | 0.2% | - | 0.2% | 0.2% |
| Benzalkonium Chloride | NF/Ph Eur | 0.01% | 0.01% | - | 0.01% | 0.02% | 0.01% | 0.01% | 0.01% | 0.01% | 0.01% | 0.01% |
| Edetate Disodium | USP | 0.05% | 0.05% | 0.05% | - | - | - | 0.05% | 0.05% | - | - | 0.05% |
| Polyvinylpyrroli done (PYPJ | USP | - | - | - | - | - | - | - | - | - | 2.0% | 2.0% |
| Sodium Phosphate Monobasic Monohydrate | USP | 0.0137% | 0.0137% | 0.0137% | 0.00171% | 0.00171% | 0.00171% | 0.00171 % | 0.00171 % | 0.001 71% | 0.00171 % | 0.00171 % |
| Sodium Phosphate Dibasic Heptahydrate | USP | 0.108% | 0.108% | 0.108% | 0.332% | 0.332% | 0.332% | 0.332% | 0.332% | 0.332% | 0.332% | 0.332% |
| Hydrochloric Acid/Sodium Hydroxide (IN stock solution) | NF | QS to pH 7.4 | QS to pH 7.4 | QS to pH 7.4 | QS to pH 7.4 | QS to pH 7.4 | QS to pH 7.4 | QS to pH 7.4 | QS to pH 7.4 | QS to pH 7.4 | QS to pH 7.4 | QS to pH 7.4 |
| Purified Water | USP/Ph Eur | QS to 100% | QS to 100% | QS to 100% | QS to 100% | QS to 100% | QS to 100% | QS to 100% | QS to 100% | QS to 100% | QS to 100% | QS to 100% |
| Expected Initial pH | | 5.5* | 5.5* | 5.5* | 7.4 | 7.4 | 7.4 | 7.4 | 7.4 | 7.4 | 7.4 | 7.4 |
| Total Buffer Concentration | | 5 mM | 5mM | 5mM | 12.5 mM | 12.5 mM | 12.5 mM | 12.5 mM | 12.5 mM | 12.5 mM | 12.5 mM | 12.5 mM |
| Expected Osmolality (mOsm/Kg) | | 369 | 314 | 369 | 314 | 314 | 314 | 314 | 314 | 314 | 314 | 314 |

| | | | | | | | | | | | | |
|---|---|---|---|---|---|---|---|---|---|---|---|---|
| *pH adjustment needed | | | | | | | | | | | | |

### Example 3

### Quantification of Impurity Concentration in Carbachol/Brimonidine Formulations After Storage

The ophthalmic formulations Formula 4, Formula 6, Formula 2, and Formula 3 comprising carbachol and brimonidine were prepared as described in Table 1 above. The samples were stored at 25 °C at 40% relative humidity (RH) or 40 °C at not more than (NMT) 25% RH. The samples were tested using the high performance liquid chromatography (HPLC) method shown in Table 3 for the presence of impurities after storage for 2, 3 and/or 5 months. The results are shown in Table 4.

**Table 3: HPLC Method**

| Column | GL Sciences Inertsil ODS-3V column, 5 µm, 4.6 x 250 mm, Part# 5020-01801 |
|---|---|
| Column Temperature | 25 °C |
| Diluent/Mobile Phase (MP) | pH 3.5 potassium octane sulfonate buffer:acetonitrile (64:36 v/v) |
| Flow Rate | 1.0 mL/min |
| Detection | UV 254 nm |
| Autosampler Temperature | Ambient |
| Injection Volume | 20 µL |
| Needle Wash | acetonitrile:water (50:50 v/v) |
| Run Time | 10 min |

**Table 4: Quantification of Impurity Concentrations in Carbachol/Brimonidine Formulations Stored for 5 Months at 25 °C and 40% RH or 40 °C and NMT 25% RH**

| Impurity | Formula 4 | | Formula 6 | | Formula 2 | | Formula 3 | |
|---|---|---|---|---|---|---|---|---|
| | 25 °C, 40% RH | 40 °C, NMT 25% RH | 25 °C, 40% RH | 40 °C, NMT 25% RH | 25 °C, 40% RH | 40 °C, NMT 25% RH | 25 °C, 40% RH | 40 °C, NMT 25% RH |
| Choline | ND | 5.1-5.4% (0.19% @3 Months) | ND | 5.0-5.4% (0.17% @3 Months) | ND | 4.7-4.8% (0.11% @3 Months) | ND | 5.9% (0.2% @3 Months) |
| Impurity B (RRT 0.67) | ND (0.09% @3 Months) | 1.2-1.3% (0.6% @3 Months) | ND (0.09% @ 3 Months) | 1.2-1.3% (0.5% @3 Months) | ND (0.08% @3 Months) | 1.5% (0.53% @3 Months) | ND (0.11% @3 Months) | 1.7% (0.58% @3 Months) |
| Impurity A (RRT 0.91) | 0.23-0.25% (0.16% @3 Months) | 2.6-2.9% (1.2% @3 Months) | 0.25% (0.16% @3 Months) | 2.3-2.9% (1.1% @3 Months) | 0.2% (0.15% @3 Months) | 2.9-3.0% (1.1% @3 Months) | 0.14-16% (0.18% @3 Months) | 3.3% (1.2% @3 Months) |
| ND = no data | | | | | | | | |

### Example 4

### Identification of an Unknown Impurity in Carbachol/Brimonidine Formulation

Three identical samples comprising carbachol and brimonidine were prepared as samples I, II, and III and were stored at 40 °C and NMT 25% relative humidity (RH) for six months and analyzed by HPLC and mass spectrometry (MS) to identify observed impurities. Brimonidine EP Impurity F Standard and EP Impurity G Standard were also analyzed by HPLC and MS for comparison to the carbachol/brimonidine formulations. The samples studied are summarized in Table 5.

**Table 5: Samples prepared for LC-PDA analysis**

| Sample Name | Stability Information | Purity |
|---|---|---|
| Carbachol/brimonidine sample (I) | 6 mo. 40 °C/NMT 25% RH | N/A |
| Carbachol/brimonidine sample (II) | 6 mo. 40 °C/NMT 25% RH | N/A |
| Carbachol/brimonidine sample (III) | 6 mo. 40 °C/NMT 25% RH | N/A |
| EP Impurity G Standard | N/A | 98.3% |
| EP Impurity F Standard | N/A | 95% |

### LC-PDA Analysis per the Method of Example 3

The mobile phase (MP), diluent and needle wash solutions were prepared per the method described in Example 3, Table 3, except the ion-pairing reagent potassium 1-octanesulfonate used in the MP was replaced with sodium 1-octanesulfonate because the potassium salt was not available. In addition, the Impurity G, Impurity F, and three carbachol/brimonidine samples described in Table 5 were prepared into solutions with the concentrations for the impurities at about 0.2 mg/mL and the samples at 0.1 mg/mL. All the solutions were analyzed by liquid chromatography photodiode array (LC-PDA) per the method in Example 3, Table 3, except the injection volume was increased from 20 µL to 30 µL for better observation of the impurity peaks. All three sample solutions showed similar chromatographic profiles to those obtained in Example 3, in which the two major impurities (RRT 0.67 and RRT 0.91) as well as the brimonidine API were observed in the chromatograms at RT 2.81 min, 4.03 min and 4.39 min, respectively; moreover, EP Impurity G was observed at the same retention time as the target impurity RRT 0.91 and EP Impurity F eluted at the same retention time as the brimonidine API. The UV spectra for all major peaks (% peak area ~0.05) in each chromatogram were extracted and examined. Since all three samples contain similar major impurities, one sample instead of three was studied in the remaining studies. Since the chromatogram for carbachol/brimonidine sample I does not have the placebo peak at RT 1.8 min, it was used for the following analysis for less matrix interference purposes. The UV spectra extracted for all the major peaks in the carbachol/brimonidine formulation sample revealed that the UV spectrum for the impurity at RRT 0.91 has unique features which include a flatter and wider second UV absorbance band at 240-260 nm and a less intensive third UV absorbance band at 320 nm. These features distinguish the impurity at RRT 0.91 from all other impurities as well as the brimonidine API, which was critical for the following LC-PDA-MS method development and analysis.

### RRT 0.91 Peak Confirmation by LC-PDA Analysis with a Low Organic MP

To further confirm the RRT 0.91 impurity observed in the above analysis was the target impurity and its UV spectrum did have those unique features mentioned above, carbachol/brimonidine sample I was prepared in 10% acetonitrile with the same concentration as used in the previous analysis, and the solution was analyzed per the same method except the MP was modified to contain lower percentage of organic (30 % mobile phase B (MPB) plus 70% mobile phase A (MPA)) and the injection volume was increased to 30 µL. The resulting 254 nm UV chromatogram shows that with the modified low organic MP, the target impurity RRT 0.91 (RT 5.62 min) separated better from the previously closely-eluted brimonidine API peak (RT 6.82 min), and a previously unobserved minor impurity which had co-eluted with either of them in the previous analysis was now observed at RT 6.37 min. However, the UV spectrum for the better-separated RRT 0.91 peak still has the similar features to what was observed previously. The special UV features (flatter and wider second UV absorbance band at 240-260 nm and a less-intensive third UV absorbance band at 320 nm) for the RRT 0.91 impurity were confirmed again.

The LC-PDA analysis with the low organic MP also confirmed that EP Impurity G was not the target impurity RRT 0.91 because the UV profiles for the two peaks are different, and their retention times are also different; the RRT 0.91 impurity elutes at RT 5.62 min, while EP Impurity G elutes at retention time 2 min with the modified mobile phase.

### LC-PDA Method Development for LC-PDA-MS Analysis

With the UV spectrum for the target impurity RRT 0.91 confirmed, a LC-MS compatible MPA (20 mM of ammonium formate solution with the pH adjusted to 3.5 by formic acid), MPB (acetonitrile) and a new diluent (10% acetonitrile in water) were prepared as an effort for LC-MS compatible method development. The sample solution concentration was kept the same as previously used; the standard solution was dilute enough not to saturate the UV detector. Injections of these solutions were performed with the new MPs through different columns including the column previously specified in Example 3, Table 3. However, all the testing results were not satisfactory since insufficient peak retention was observed due to no ion pairing reagent in the MPs. Finally, a Phenomenex Kinetex XB C-18 column (2.6 µm, 4.6 x 100 mm, Part# 00F-4496-E0) was tried for the analysis and good retention and separation of the target RRT 0.91 peak was achieved. The final developed LC-PDA method is summarized in Table 6. With this method, the standard and sample solutions prepared above were analyzed. In the resulting chromatogram the RRT 0.91 impurity elutes at RT 3.4 min while the brimonidine API elutes out at RT 2.1 min, confirming a very good separation between the two. The extracted UV spectrum for the RRT 0.91 is also consistent to what was observed previously.

**Table 6: LC-PDA-TOFMS conditions used for identification analysis**

| | Column | Phenomenex Kinetex XB C-18 column , 2.6 µm, 4.6 x 100 mm, Part# 00F-4496-E0 |
|---|---|---|
| | Column Temperature | 25 °C |
| | Mobile Phase A (MPA) | pH 3.5 20 mM ammonium formate buffer |
| HPLC | Mobile Phase B (MPB) | Degassed acetonitrile |
| | Standard Diluent | 10% acetonitrile |
| | Injection Volume | 30 µL |
| | Needle Wash | 50:50 acetonitrile:water v/v |
| | Run Time | 10 min |
| | Flow Rate | 0.8 mL/min |
| | Elution | Isocratic 20% MPB + 80% MPA |
| | Autosampler Temperature | Ambient |
| | Ionization | Positive ESI |
| | Nebulizing Gas | Nitrogen (35 psig) |
| MS | Drying Gas | Nitrogen (325 °C, 11 L/min) |
| | V_{frag} | 100 volts |
| | V_{cap} | 3500 volts |
| | Mass Scan Rate | 1 spectra/s |
| | Scan Range | 110-950 m/z |
| | Time window for LC stream to enter MSD | 3.1-4.5 min |

### LC-PDA-MS Analysis

With the LC-PDA method developed in the previous section plus the mass spectrometric parameters developed accordingly (bottom half of Table 6), EP Impurity G, EP Impurity F, and carbachol/brimonidine sample A solutions were analyzed by LC-PDA-MS. The target impurity (RRT 0.91) was observed in the UV chromatogram at RT 3.5 min and in the total ion chromatogram at RT 3.57 min. Extraction of these two peaks resulted in the UV and mass spectra, which revealed a clear monoisotopic [M+H]⁺ peak at m/z 335.0252; high resolution mass matching indicated it has a molecular formula of C₁₂H₁₁BrN₆O with a mass matching error of only 0.48 ppm, as shown in Table 7. Compared to brimonidine API (C₁₁H₁₀BrN₅), the former has an extra CONH group in its molecule.

**Table 7: High Resolution Mass Spectral Analysis Results for RRT 0.91 Impurity**

| Impurity Name | Monoisotopic Ion found (m/z) | Ion Form | Theoretical Monoisotopic Mass for M | Molecular Formula for M | Absolute Mass Matching Error (ppm) | Overall Mass Match Score Generated by Masshunter Software (%) |
|---|---|---|---|---|---|---|
| Impurity A (RRT 0.91) | 335.0252 | [M+H]⁺ | 334.0178 Da | C₁₂H₁₁BrN₆O | 0.48 | 96.75 |

### Example 5

### Forced Degradation Study of Carbachol/Brimonidine Formulation

### Materials

The materials used in this forced degradation study are shown in Table 8 below.

**Table 8: Materials Used for Forced Degradation Studies**

| **Material Name** |
|---|
| Brimonidine tartrate API |
| Carbachol API |
| Carbachol/brimonidine formulation (6 month accelerated stability sample) |
| Drug Product Vehicle |

### Stress Sample Solutions Preparation (Phase I)

Following the procedures shown in Table 9, the two APIs combined were stressed with moisture and heat (Sample A), 0.1 M HCl at room temperature (Sample B), 0.1 M NaOH at room temperature (Sample C), vehicle and heat (Sample D), 1 M HCl at room temperature (Sample E), and 1 M NaOH at room temperature (Sample F) for 66 hours. A drug product vehicle was stressed under heat for 66 hours as a control (Sample G). After the stress treatment was completed, all samples were removed from condition, allowed to cool to room temperatures, then neutralized where appropriate, and brought to a similar concentration level. The samples were capped and shaken until all the materials in each sample were dissolved. Then each sample solution was diluted in the HPLC method diluent used in Example 4, Table 6 and mixed well. For retention time verification purposes, a reference sample solution was also prepared using a carbachol/brimonidine six month accelerated stability sample (Table 8). The brimonidine tartrate analytical concentration for all of the degraded and the reference sample solutions was 0.1 mg/mL.

**Table 9: Forced Degradation Sample Preparations (Phase I)**

| Sample | VF Volume | APIs Added* | Stress Conditions | Procedure |
|---|---|---|---|---|
| A | 50 mL | 5.0 mg of brimonidine tartrate and 137.5 mg of carbachol | Moisture and heat (50 °C) | Added 5.0 mL of water into the flask containing both APIs, capped, and put in a 50 °C oven for 66 hours |
| B | 50 mL | 5.0 mg of brimonidine tartrate and 137.5 mg of carbachol | 0.1 M HCl, room temperature | Added 5.0 mL of 0.1 M HCl into the flask containing both APIs, capped, and put on a stir plate with the solution stirred under room temperature for 66 hours |
| C | 50 mL | 5.0 mg of brimonidine tartrate and 137.5 mg of carbachol | 0.1 M NaOH, room temperature | Added 5.0 mL of 0.1 M NaOH into the flask containing both APIs, capped, and put on a stir plate with the solution stirred under room temperature for 66 hours |
| D | 50 mL | 5.0 mg of brimonidine tartrate and 137.5 mg of carbachol | Vehicle and heat (50 °C) | Added 1.0 mL of a vehicle into the flask containing both APIs, capped, and put in a 50 °C oven for 66 hours |
| E | 50 mL | 5.0 mg of brimonidine tartrate and 137.5 mg of carbachol | 1 M HCl, room temperature | Added 5.0 mL of 1 M HCl into the flask containing both APIs, capped, and put on a stir plate with the solution stirred under room temperature for 66 hours |
| F | 50 mL | 5.0 mg of brimonidine tartrate and 137.5 mg of carbachol | 1 M NaOH, room temperature | Added 5.0 mL of 1 M NaOH into the flask containing both APIs, capped, and put on a stir plate with the solution stirred under room temperature for 66 hours |
| G | 10 mL | None | Vehicle, heat (50 °C, as control) | Added 0.2 mL of a vehicle into the flask containing no APIs, capped, and put in a 50 °C oven for 66 hours |

| | | | | |
|---|---|---|---|---|
| *The APIs w/w ratio was the same as that in Table 8. | | | | |

### LC-PDA Analysis of Forced Degradation Sample Solutions

All of the sample solutions prepared as described in in Table 9 were analyzed per the HPLC-PDA method shown in Example 4, Table 6. The RRT 0.91 impurity was observed at RT 3.4 min only in the reference sample and in the base-stressed samples (Sample C, Sample F, and Sample G). The level of the impurity present in both base-stressed samples is about 0.02% relative to that of the brimonidine peak. The extracted UV spectra for the RRT 0.91 impurity from the carbachol/brimonidine formulation (6 month accelerated stability sample) and the two base-stressed samples are similar for each.

### Mass Verification by HPLC-MS for the RRT 0.91 Impurity (Phase I)

The reference sample solution and the two base-stressed combined API sample solutions were analyzed by HPLC-PDA-MS using the method shown in Example 4, Table 6. In the resulting chromatograms, the RRT 0.91 ion peak was clearly observed at RT 3.63 min for each sample. The extracted mass spectra for the ion peak in all three samples look similar and confirmed that the RRT 0.91 peak has a molecular formula of C₁₂H₁₁BrN₆O.

### Stress Sample Solution Preparations (Phase II)

The Phase I forced degradation study confirmed that the RRT 0.91 impurity was formed when the two APIs together were stressed under basic (either 0.1 M NaOH or 1 M NaOH) conditions and at room temperatures. To study temperature impact on the reaction, the two APIs, brimonidine tartrate and carbachol, were combined and stressed under both base and heat (45 °C) conditions simultaneously.

Additionally, to further confirm that the presence of both APIs was needed for the formation of the RRT 0.91 peak, individual API samples were also stressed in the same way. Table 10 shows the degradation sample preparation procedures used. After the stress treatment was completed, all samples (H-M) were removed from condition, allowed to cool to room temperatures, neutralized where appropriate, and brought to a similar concentration level. The samples were capped and shaken until all the materials in each flask were dissolved. Then each sample solution was diluted in the HPLC method diluent (Example 4, Table 6) and mixed well.

**Table 10: Forced Degradation Sample Preparations (Phase II)**

| Sample | APIs Used | Stress Conditions | Procedure |
|---|---|---|---|
| H | 5.0 mg of brimonidine tartrate | 0.1 M NaOH and heat (45 °C) | Added 5.0 mL of 0.1 M NaOH into the flask containing brimonidine tartrate, shook to disperse the material, added in a small stir bar, capped, and put on a 45 °C hot plate with the solution stirred at 50 rpm for 66 hours |
| I | 5.0 mg of brimonidine tartrate | 1 M NaOH and heat (45 °C) | Added 5.0 mL of 1 M NaOH into the flask containing brimonidine tartrate, shook to disperse the material, added in a small stir bar, capped, and put on a 45 °C hot plate with the solution stirred at 50 rpm for 66 hours |
| J | 137.5 mg of carbachol | 0.1 M NaOH and heat (45 °C) | Added 5.0 mL of 0.1 M NaOH into the flask containing carbachol, shook to disperse the material, added in a small stir bar, capped, and put on a 45 °C hot plate with the solution stirred at 50 rpm for 66 hours |
| K | 137.5 mg of carbachol | 1 M NaOH and heat (45 °C) | Added 5.0 mL of 1 M NaOH into the flask containing carbachol, shook to disperse the material, added in a small stir bar, capped, and put on a 45 °C hot plate with the solution stirred at 50 rpm for 66 hours |
| L | 5.0 mg of brimonidine tartrate and 137.5 mg of carbachol | 0.1 M NaOH and heat (45 °C) | Added 5.0 mL of 0.1 M NaOH into the flask containing both APIs, shook to disperse the material, added in a small stir bar, capped, and put on a 45 °C hot plate with the solution stirred at 50 rpm for 66 hours |
| M | 5.0 mg of brimonidine tartrate and 137.5 mg of carbachol | 1 M NaOH and heat (45 °C) | Added 5.0 mL of 1 M NaOH into the flask containing both APIs, shook to disperse the material, added in a small stir bar, capped, and put on a 45 °C hot plate with the solution stirred at 50 rpm for 66 hours |

### LC-PDA Analysis of the Base and Heat-Stressed Sample Solutions

All the base and heat-stressed sample solutions prepared as described in Table 10 were analyzed by LC-PDA analysis per the method shown in Example 4, Table 6. In the resulting 254 nm UV chromatograms, the target impurity RRT 0.91 was observed in the heat and base-stressed sample solutions when both APIs were present, but not in the base and heat-stressed individual API solutions. This further confirmed that the RRT 0.91 impurity was a reaction product of the two APIs.

Table 11 shows the relative% peak area (versus brimonidine API peak) for the RRT 0.91 peak in each base-stressed combined APIs with or without heating involved. The higher % peak area values for the impurity in both heat and base-stressed samples indicates that heat accelerates the formation of RRT 0.91 impurity under basic conditions.

**Table 11: Comparison of Relative % Peak Area for RRT 0.91 Impurity Formed Under Basic Conditions Between Room Temperatures and Higher Temperature at 45 °C**

| Stress Conditions | Relative % Peak Area for RRT 0.91 Impurity (Versus Peak Area of Brimonidine) |
|---|---|
| 0.1 M NaOH, room temperature | 0.02% |
| 1 M NaOH, room temperature | 0.02% |
| 0.1 M NaOH, 45 °C | 0.15% |
| 1 M NaOH, 45 °C | 0.03% |

### HPLC-PDA Analysis of All Stressed Sample Solutions per Prototype Method

A new prototype HPLC method has been developed to study early eluting peaks not well separated by the method described in Example 3, Table 3. The method better separates early eluting brimonidine-related impurities. The conditions are shown in Table 12. All sample solutions from the Phase I and II stress studies were analyzed with the prototype method. In the resulting 254 nm chromatograms, the RRT 0.91 impurity was observed in the chromatograms at RT 13.4 minutes for base-stressed combined APIs with or without heating; but not in other stressed samples that did not contain base. This was consistent with the analysis results per the method described in Example 4, Table 6. Another unknown impurity, impurity B, was also observed in the chromatogram for the 0.1 M NaOH and heat-stressed combined API sample at RT 3.44 min with a level of ~0.05 % relative to the brimonidine peak area. The extracted UV spectrum for impurity B is consistent with the UV Spectrum previously observed in stability samples. Impurity B was also observed in other base-stressed combined APIs, but in very low levels.

**Table 12: Prototype HPLC Method**

| Column | GL Sciences Inertsil ODS-3V column, 5 µm, 4.6 x 250 mm, Part# 5020-01801 |
|---|---|
| Column Temperature | 25 °C |
| Diluent/Mobile Phase (MP) | pH 3.5 sodium octane sulfonate buffer:acetonitrile (80:20 v/v) |
| Flow Rate | 2.0 mL/min |
| Detection | UV 254 nm |
| Autosampler Temperature | Ambient |
| Injection Volume | 20 µL |
| Needle Wash | acetonitrile:water (50:50 v/v) |
| Run Time | 30 min |

### Example 6

### Additional Exemplary Carbachol/Brimonidine Formulations

Ophthalmic formulations comprising carbachol and brimonidine are prepared by dissolving the components in aqueous solution at the concentration ranges shown in Table 13. Opthalmic formulations which do not fall within the scope of the claims are described for reference only.

**Table 13: Exemplary Carbachol/Brimonidine Formulations**

| Component | Formula 22 | Formula 23 | Formula 24 | Formula 25 | Formula 26 | Formula 27 |
|---|---|---|---|---|---|---|
| Carbachol | 2.25 wt % - 3 wt % | 2.25 wt % - 3 wt % | 2.25 wt % - 3 wt % | 2.25 wt % - 3 wt % | 2.25 wt % - 3 wt % | 2.25 wt % - 3 wt % |
| Brimonidine | 0.1 wt % to 0.3 wt % | 0.1 wt % to 0.3 wt % | 0.1 wt % to 0.3 wt % | 0.1 wt % to 0.3 wt % | 0.1 wt % to 0.3 wt % | 0.1 wt % to 0.3 wt % |
| Sodium Carboxymethyl Cellulose | 0.8 wt % to 1.2 wt % | 0.8 wt % to 1.2 wt % | 0.8 wt % to 1.2 wt % | - | - | - |
| Hydroxypropylmethyl cellulose | - | - | - | 0.1 wt % to 1.2 wt % | 0.1 wt % to 1.2 wt % | 0.1 wt % to 1.2 wt % |
| Benzalkonium Chloride | 0.0075 wt % to 0.025 wt % | 0.0075 wt % to 0.025 wt % | - | 0.0075 wt % to 0.025 wt % | 0.0075 wt % to 0.025 wt % | - |
| Polyvinylpyrrolidone (PVP) | - | 1.5 wt % to 2.5 wt % | - | - | 1.5 wt % to 2.5 wt % | - |
| Phosphate buffer* | 0.1 wt % to 0.2 wt % | 0.1 wt % to 0.2 wt % | 0.1 wt % to 0.2 wt % | 0.1 wt % to 0.2 wt % | 0.1 wt % to 0.2 wt % | 0.1 wt % to 0.2 wt % |
| Purified water | QS to 100% | QS to 100% | QS to 100% | QS to 100% | QS to 100% | QS to 100% |

| | | | | | | |
|---|---|---|---|---|---|---|
| *total concentration of phosphate buffer comprising sodium phosphate monobasic monohydrate and sodium phosphate dibasic heptahydrate | | | | | | |

Additional ophthalmic formulations comprising carbachol and brimonidine are prepared by dissolving the components in aqueous solution at the concentrations shown in Table 14. Opthalmic formulations which do not fall within the scope of the claims are described for reference only.

**Table 14: Additional exemplary Carbachol/Brimonidine Formulations**

| Component | Formula 28 | Formula 29 | Formula 30 | Formula 31 | Formula 32 | Formula 33 |
|---|---|---|---|---|---|---|
| Carbachol | 2.75 wt % | 2.75 wt % | 2.75 wt % | 2.75 wt % | 2.75 wt % | 2.75 wt % |
| Brimonidine | 0.1 wt % | 0.1 wt % | 0.1 wt % | 0.1 wt % | 0.1 wt % | 0.1 wt % |
| Sodium Carboxymethyl Cellulose | 1 wt % | 1 wt % | 1 wt % | - | - | - |
| Hydroxypropylmethyl cellulose | - | - | - | 0.2 wt % | 0.2 wt % | 0.2 wt % |
| Benzalkonium Chloride | 0.01 wt % | 0.01 wt % | - | 0.01 wt % | 0.01 wt % | - |
| Polyvinylpyrrolidone (PVP) | - | 2 wt % | - | - | 2 wt % | - |
| Phosphate buffer* | 0.12 wt % | 0.12 wt % | 0.12 wt % | 0.12 wt % | 0.12 wt % | 0.12 wt % |
| Purified water | QS to 100% | QS to 100% | QS to 100% | QS to 100% | QS to 100% | QS to 100% |

| | | | | | | |
|---|---|---|---|---|---|---|
| *total concentration of phosphate buffer comprising sodium phosphate monobasic monohydrate and sodium phosphate dibasic heptahydrate | | | | | | |

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims.

Citation or identification of any reference in this application shall not be construed as an admission that such reference is available as prior art to the present invention. To the extent that section headings are used, they should not be construed as necessarily limiting.

## Claims

1. An ophthalmic formulation comprising 2.75 wt% carbachol, or a pharmaceutically acceptable salt thereof, 0.1 wt% brimonidine, or a pharmaceutically acceptable salt thereof, and 0.2 wt% hydroxypropylmethyl cellulose (HPMC), and from 0.05 wt % to 1 wt % of one or more buffers, wherein the pH is 7.4, and
wherein the formulation comprises:
(i) less than 5 wt % impurity A after storage at 40 °C and not more than (NMT) 25% relative humidity for 5 months, wherein impurity A has the structure: 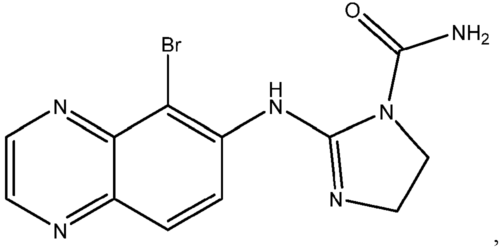 or a tautomer thereof, wherein the concentration of impurity A is measured by high-performance liquid chromatography (HPLC) in pH 3.5 potassium octane sulfonate buffer:acetonitrile (64:36 v/v) and impurity A has a relative retention time (RRT) of 0.9 relative to brimonidine; and/or
(ii) less than 2 wt % impurity B after storage at 40 °C and not more than (NMT) 25% relative humidity for 5 months, wherein impurity B has the structure: 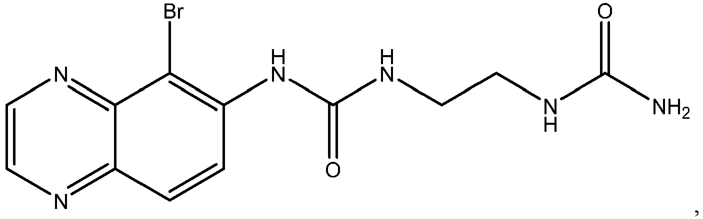 or a tautomer thereof, wherein the concentration of impurity B is measured by high-performance liquid chromatography (HPLC) in pH 3.5 potassium octane sulfonate buffer:acetonitrile (64:36 v/v) and impurity B has a relative retention time (RRT) of 0.67 relative to brimonidine.

2. The ophthalmic formulation of claim 1, wherein the ophthalmic formulation does not contain benzalkonium chloride (BAK).

3. The ophthalmic formulation of claim 1 or 2, wherein the formulation does not contain etheylenediaminetetraacetic acid (EDTA).

4. The ophthalmic formulation of any one of claims 1 to 3, wherein the one or more buffers is a phosphate buffer.

5. The ophthalmic formulation of claim 4, wherein the phosphate buffer comprises sodium phosphate monobasic monohydrate and sodium phosphate dibasic heptahydrate.

6. The ophthalmic formulation of any one of claims 1-5, wherein the brimonidine is brimonidine tartrate.

7. The ophthalmic formulation of any one of claims 1-6 for use in ameliorating or reducing presbyopia in at least one eye of a subject.

8. The ophthalmic formulation for use according to claim 7, wherein the amelioration or reduction of presbyopia is effective for at least 9 hours.

## Patentansprüche

1. Ophthalmische Formulierung, die 2,75 Gew.-% Carbachol oder eines pharmazeutisch unbedenklichen Salzes davon, 0,1 Gew.-% Brimonidin oder eines pharmazeutisch unbedenklichen Salzes davon und 0,2 Gew.- % Hydroxypropylmethylcellulose (HPMC) sowie 0,05 Gew.-% bis 1 Gew.-% eines oder mehrerer Puffer umfasst, wobei der pH-Wert 7,4 beträgt, und
wobei die Formulierung Folgendes umfasst:
(i) weniger als 5 Gew.-% Verunreinigung A nach Lagerung bei 40 °C und nicht mehr als (NMT) 25 % relativer Feuchtigkeit über 5 Monate, wobei Verunreinigung A die folgende Struktur aufweist: 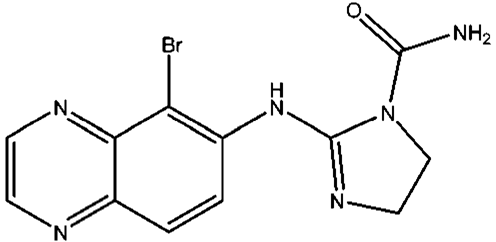 oder ein Tautomer davon ist, wobei die Konzentration der Verunreinigung A durch Hochleistungsflüssigkeitschromatographie (HPLC) in Kaliumoctansulfonatpuffer pH 3,5:Acetonitril (64:36 v/v) gemessen wird und die Verunreinigung A eine relative Retentionszeit (RRT) von 0,9 relativ zu Brimonidin aufweist, und/oder
(ii) weniger als 2 Gew.-% Verunreinigung B nach Lagerung bei 40 °C und nicht mehr als (NMT) 25 % relativer Feuchtigkeit über 5 Monate, wobei Verunreinigung B die folgende Struktur aufweist: 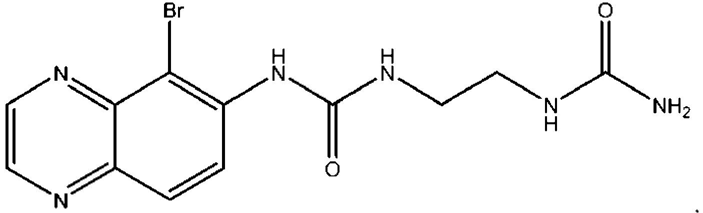 oder ein Tautomer davon ist, wobei die Konzentration der Verunreinigung B durch Hochleistungsflüssigkeitschromatographie (HPLC) in Kaliumoctansulfonatpuffer pH 3,5:Acetonitril (64:36 v/v) gemessen wird und die Verunreinigung B eine relative Retentionszeit (RRT) von 0,67 relativ zu Brimonidin aufweist.

2. Ophthalmische Formulierung nach Anspruch 1, wobei die ophthalmische Formulierung kein Benzalkoniumchlorid (BAK) enthält.

3. Ophthalmische Formulierung nach Anspruch 1 oder 2, wobei die Formulierung keine Ethylendiamintetraessigsäure (EDTA) enthält.

4. Ophthalmische Formulierung nach einem der Ansprüche 1 bis 3, wobei es sich bei dem einen oder den mehreren Puffern um einen Phosphatpuffer handelt.

5. Ophthalmische Formulierung nach Anspruch 4, wobei der Phosphatpuffer Natriumdihydrogenphosphat-monohydrat und Dinatriumhydrogenphosphat-heptahydrat umfasst.

6. Ophthalmische Formulierung nach einem der Ansprüche 1-5, wobei es sich bei dem Brimonidin um Brimonidintartrat handelt.

7. Ophthalmische Formulierung nach einem der Ansprüche 1-6 zur Verwendung bei der Linderung oder Verringerung von Presbyopie in mindestens einem Auge eines Subjekts.

8. Ophthalmische Formulierung zur Verwendung nach Anspruch 7, wobei die Linderung oder Verringerung von Presbyopie mindestens 9 Stunden wirksam ist.

## Revendications

1. Formulation ophtalmique comprenant 2,75 % en poids de carbachol, ou un sel pharmaceutiquement acceptable de celui-ci, 0,1 % en poids de brimonidine, ou un sel pharmaceutiquement acceptable de celle-ci, et 0,2 % en poids d'hydroxypropylméthylcellulose (HPMC), et de 0,05 % en poids à 1 % en poids d'un ou plusieurs tampons, dans laquelle le pH est de 7,4, et
la formulation comprenant :
(i) moins de 5 % en poids d'impureté A après stockage à 40 °C et pas plus de (NMT) 25 % d'humidité relative pendant 5 mois, l'impureté A ayant la structure : 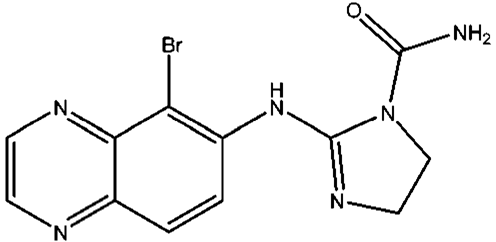 ou une forme tautomère de celle-ci, la concentration de l'impureté A étant mesurée par chromatographie en phase liquide à haute performance (HPLC) dans un tampon octane sulfonate de potassium à pH 3,5:acétonitrile (64:36 v/v) et l'impureté A ayant un temps de rétention relatif (RRT) de 0,9 par rapport à la brimonidine ; et/ou
(ii) moins de 2 % en poids d'impureté B après stockage à 40 °C et pas plus de (NMT) 25 % d'humidité relative pendant 5 mois, l'impureté B ayant la structure : 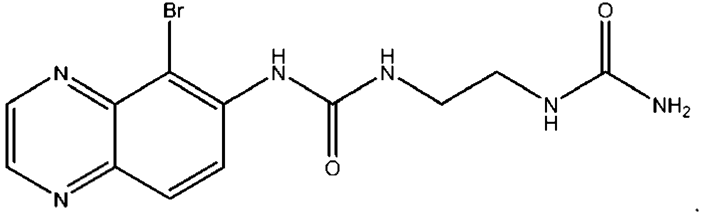 ou une forme tautomère de celle-ci, la concentration d'impureté B étant mesurée par chromatographie en phase liquide à haute performance (HPLC) dans un tampon octane sulfonate de potassium à pH 3,5:acétonitrile (64:36 v/v) et l'impureté B ayant un temps de rétention relatif (RRT) de 0,67 par rapport à la brimonidine.

2. Formulation ophtalmique selon la revendication 1, dans laquelle la formulation ophtalmique ne contient pas de chlorure de benzalkonium (BAK).

3. Formulation ophtalmique selon la revendication 1 ou 2, dans laquelle la formulation ne contient pas d'acide éthylènediaminetétraacétique (EDTA).

4. Formulation ophtalmique selon l'une quelconque des revendications 1 à 3, dans laquelle le ou les tampons est/sont un tampon phosphate.

5. Formulation ophtalmique selon la revendication 4, dans laquelle le tampon phosphate comprend un monohydrate de phosphate de sodium monobasique et un heptahydrate de phosphate de sodium dibasique.

6. Formulation ophtalmique selon l'une quelconque des revendications 1 à 5, dans laquelle la brimonidine est le tartrate de brimonidine.

7. Formulation ophtalmique selon l'une quelconque des revendications 1-6 destinée à être utilisée pour améliorer ou réduire la presbytie dans au moins un œil d'un sujet.

8. Formulation ophtalmique destinée à être utilisée selon la revendication 7, dans laquelle l'amélioration ou la réduction de la presbytie est efficace pendant au moins 9 heures.
